# EUROPEAN PATENT APPLICATION

(11) **EP 2 518 141 A1**
(43) Date of publication of application: **31.10.2012**
(21) Application number: 12170345.8
(22) Date of filing: 01.08.2008
(51) Int. Cl.: C12N 5/079, A61K 35/30

(54) **Stem cells derived from the carotid body and uses thereof**

(30) Priority: 02.08.2007 ES 200702167
(62) Divisional of application: 08786807.1
(71) Applicant: Universidad de Sevilla, 41012 Sevilla (ES)
(72) Inventor: Lopez Barneo, Jose, 41012 Sevilla (ES); Pardal, Ricardo, 41012 Sevilla (ES); Ortega-Saenz, Patricia, 41012 Sevilla (ES); Duran, Rocio, 41012 Sevilla (ES); Bonilla Henao, Victoria Eugenia, 41012 Sevilla (ES); Ordonez Fernandez, Antonio, 41012 Sevilla (ES); Toledo Aral, Juan Jose, 41012 Sevilla (ES)
(74) Representative: Goodfellow, Hugh Robin

(57) **Abstract**

Adult stem cells obtained from the carotid body, characterized in that they are positive for the phenotypic marker GFAP (glial fibrillary acidic protein) and negative for the phenotypic markers TH (tyrosine hydroxylase) and nestin, are described. These stem cells can undergo proliferation, self-renewal and differentiation to progenitor cells and differentiated cells. Said stem cells, progenitor cells and differentiated cells, expanded by any method, can be used in the treatment of neurodegenerative diseases such as Alzheimer's disease or Parkinson's disease.

## Description

### FIELD OF THE INVENTION

The present invention relates to the isolation and characterization of stem cells derived from the carotid body (CB), to methods for their expansion and differentiation, and to their potential applications both in research and in therapy, for example in the treatment of neurodegenerative diseases such as Alzheimer's disease or Parkinson's disease.

### BACKGROUND OF THE INVENTION

Neural or paraneural cells that secrete neurotransmitters, neuromodulators or enzymes have been employed for the treatment of neurodegenerative diseases, as an alternative to drug therapy. These cells have been used experimentally, although with limited success so far, in clinical studies.

At the end of the 1970s and beginning of the 1980s, the beneficial effect of intrastriatal transplants of adrenal medulla, which possesses dopamine-secreting chromaffin cells, was observed in experimental models of Parkinson's disease in rats.

The therapeutic effect of paraneural adrenal medulla autografts in Parkinson's disease patients was investigated in the 1980s. These transplants showed little functionality and early rejection. The beneficial effect of transplants of neural cells obtained from fetalsubstantianigra, using the experimental model of Parkinson's disease in rodents, was observed at the beginning of the 1980s. The functionality and survival of these transplants is better than that of transplants of chromaffin adrenal cells, but their clinical use is restricted to just certain countries owing to ethical or legal criteria. Moreover, the results obtained in double-blind trials in patients are less favourable than expected and in some cases the implantation of fetal cells produces undesirable motor complications. The use of xenografts with fetal cells of nonhuman substantianigra, for example from the pig, is being tested clinically. However, this technology has some very important limitations, such as strong immunological rejection and the possible transfer of infectious pathogens between species.

The carotid body is a small cluster of chemoreceptors and supporting cells located near the bifurcation of the carotid artery. It is made up of two types of cell: type I (glomus) cells, and type II (sustentacular) cells. Glomus cells are derived from neural crest, which, in turn are derived from neuroectoderm. They release a variety of neurotransmitters, including acetylcholine, ATP, and dopamine that trigger excitatory postsynaptic potentialsin synapsed neurons leading to the respiratory center. Type II cells resemble glia, express the glial marker S100 and act as supporting cells.

At the end of the 1990s aggregates of carotid body glomus cells were developed for the treatment of Parkinson's disease in models of the disease in rodents (Espejo et al., 1998; Neuron 20, 197-206). Later it was confirmed that the technique was feasible in a primate model (Luquin et al., 1999; Neuron 22, 743-750). Finally the first clinical trials with Parkinson's disease patients were conducted (Arjona et al., 2003; Neurosurgery 53, 321-328), where the improvement was always partial due probably and principally to the small amount of glomus tissue available for transplantation.

Cellular therapy, based on the principle of the regenerative capacity of dopaminergic cells from the application of stem cells, represents a promising therapeutic route in the treatment of neurodegenerative diseases. One of the main aims of research in this field is to identify the best type of stem cell that can be applied safely and effectively in the treatment of neurodegenerative diseases. The type of stem cell suitable for the treatment of neurodegenerative diseases should be capable of sufficient expansion, with capacity for differentiation to specific cells (e.g. dopaminergic cells) that are fully functional, integration in nerve tissue and possibly, though not necessarily, establishing electrochemical contact with the adjacent cells, and lastly, its application should not be limited by questions of immunological rejection nor by ethical considerations.

In this connection, US patent application US 2003/0095956 describes the use of a population of undifferentiated neural cells that includes cells with a GFAP-/nestin+ phenotype, obtained from mouse embryo brain and striatum and from juvenile and adult mouse brain tissue potentially useful in the treatment of neurodegenerative diseases, e.g. Parkinson's disease. However, these cells are different from the nestin+ cells provided by the present invention, since the latter come from the carotid body (and not from the brain or from the striatum) and display the property of differentiating to glomus cells (TH+ and sensitive to hypoxia and hypoglycaemia), a property that the nestin+ precursors isolated from the brain or from the striatum do not have. Moreover, international patent application WO 00/29549 describes a method of cultivating stem cells from the neural crest (NCSC) that comprises cultivating said NCSC in conditions of hypoxia; said NCSC are potentially useful in the treatment of neurodegenerative diseases associated with deficiency of a neurotransmitter, e.g. Parkinson's disease or Alzheimer's disease. The cells provided by this invention are different cells from those defined in said patent application WO 00/29549; furthermore, in this invention hypoxia is used for stimulating the proliferation of the cells from the CB because it has long been known that hypoxia stimulates growth of the CB in *vivo.*

Although there has been considerable progress in understanding of the treatment of neurodegenerative diseases by cellular therapy, no population of adult stem cells has been found that can be used safely and effectively in the treatment of said neurodegenerative diseases. Said cell population would provide a simple and effective method for repairing the damage caused by the neurodegenerative diseases. Accordingly, it is still necessary to improve the efficacy of the existing treatments, as well as search for alternatives that can repair the damage caused by the neurodegenerative diseases.

In addition, there is still a need to isolate, cultivate, maintain, propagate and differentiate adult stem cells for developing cell types intended for different applications. Said applications may include the use of autologous stem cells for treating diseases, for example neurodegenerative diseases, heart diseases, etc.

### SUMMARY OF THE INVENTION

Now, the inventors discovered, surprisingly, the presence of stem cells in the adult peripheral nervous system (PNS), more concretely in the carotid body (CB). Said adult stem cells, contrary to what might be thought initially, are not glomus cells (or type I cells), but sustentacular cells or type II cells. As shown in the examples in this description, said adult stem cells have the capacity to divide (proliferation), renew themselves by means of cell division over long periods of time (self-renewal) and, under certain physiological or experimental conditions, the capacity to be induced to differentiate into other types of specific differentiated cells (differentiation). Moreover, the inventors developed an *in-vitro* method for the expansion of the CB that makes it possible to obtain cellular aggregates capable of expressing and secreting trophic factors, neurotransmitters, neuromodulators, enzymes, etc. Transplants with said adult stem cells or cellular aggregates obtained by said method of expansion could be useful in clinical practice, in particular in the treatment of neurodegenerative diseases.

As already mentioned in the Background of the Invention, autografts with cells obtained from the carotid body for the treatment of neurodegenerative diseases have been used for the treatment of degenerative diseases, since it is known that in these pathologies, deficiency of some molecules (for example, neurotransmitters, growth factors, etc.) can be compensated by implanting cells capable of expressing and secreting said molecules. In any case, autografts with glomus cells obtained from the CB have proved inadequate for mitigating the damage caused by diseases, such as Alzheimer's disease or Parkinson's disease, therefore it is necessary to improve said treatments or find alternatives to them. The experiments described in detail below show that the glomus cells obtained by the method provided by this invention, as well as being capable of obtaining a larger amount of them, makes it possible to improve the methodologies of treatment by autografts of cells from the CB that are currently in existence.

Therefore, in one aspect, the present invention relates to an isolated adult stem cell, obtained from the CB, called "stem cell of the invention" hereinafter, characterized in that it is positive for the phenotypic marker GFAP (glial fibrillary acidic protein; EntrezGeneID2670) and negative for the phenotypic markers TH (tyrosine hydroxylase; EntrezGeneID: 7054) and nestin (EntrezGeneID: 10763).

In another aspect, the invention relates to an isolated adult progenitor cell, derived from said stem cell of the invention, called "progenitor cell of the invention" hereinafter, characterized in that it is positive for the phenotypic marker nestin. In a particular embodiment said progenitor cell of the invention is slightly or weakly positive for the phenotypic marker GFAP and positive for the phenotypic marker nestin. In another particular embodiment, said progenitor cell of the invention is negative for the phenotypic marker GFAP and positive for the phenotypic marker nestin.

In another aspect, the invention relates to an adult differentiated cell, derived from any of the aforesaid cell types (i.e. from the stem cell of the invention or from the progenitor cell of the invention), called "differentiated cell of the invention" hereinafter, characterized in that it is negative for the phenotypic marker nestin and is positive for a specialization marker; in a particular embodiment, said specialization marker is tyrosine hydroxylase (TH) or smooth muscle actin (SMA).

In another aspect, the invention relates to a reversibly immortalized cell, called "immortalized cell of the invention" hereinafter, said cell being a stem cell of the invention, a progenitor cell of the invention or a differentiated cell of the invention.

In another aspect, the invention relates to an isolated population of cells derived from CB, called "cell population of the invention" hereinafter, that comprises a set of cells selected from the group comprising the stem cells of the invention, the progenitor cells of the invention, the differentiated cells of the invention, the immortalized cells of the invention, and combinations thereof.

In another aspect, the invention relates to a method for the expansion of said stem cells of the invention or of said progenitor cells of the invention, that comprises cultivating said stem cells or progenitor cells of the invention under conditions of hypoxia in a culture medium specific to the neural crest.

In another aspect, the invention relates to a pharmaceutical composition, called pharmaceutical composition of the invention hereinafter, that includes a therapeutically effective amount of said stem cells, progenitor cells or differentiated cells of the invention, or combinations thereof, or of said immortalized cells of the invention, or of said cell population of the invention, and a pharmaceutically acceptable excipient.

In another aspect, the invention relates to the use of said stem cells, progenitor cells or differentiated cells of the invention or combinations thereof, or of said immortalized cells of the invention, or of said cell population of the invention, in the development of a pharmaceutical composition for the treatment of a neurodegenerative disease, for example in the treatment of Alzheimer's disease, in the treatment of Parkinson's disease, in the treatment of ischaemic lesions of the nervous system, in the treatment of traumatic lesions of the nervous system, or in the treatment of autoimmune lesions of the nervous system.

In another aspect, the invention relates to a method of treatment of a disease, preferably a neurodegenerative disease or a disease caused by an ischaemic, traumatic or autoimmune lesion of the nervous system that comprises grafting (implanting or transplanting) in the regions affected by said disease, a therapeutically effective amount of said stem cells, progenitor cells or differentiated cells of the invention or combinations thereof, or of said immortalized cells of the invention, or of said cell population of the invention, preferably differentiated cells of the invention, in particular glomus cells (nestin-/TH+), preferably in the form of neurospheres.

In another aspect, the invention relates to a method for *in-vitro* evaluation of the cellular response to a potentially therapeutic compound that comprises contacting a culture that contains stem cells, progenitor cells or differentiated cells of the invention or combinations thereof, and/or immortalized cells of the invention, and/or a cell population of the invention with a potentially therapeutic compound and evaluating the effects caused by said compound on the cells present in said culture.

In another aspect, the invention relates to the use of said stem cells, progenitor cells or differentiated cells of the invention or combination thereof, or of said immortalized cells of the invention, or of said cell population of the invention, for the production of GDNF.

### BRIEF DESCRIPTION OF THE DRAWINGS

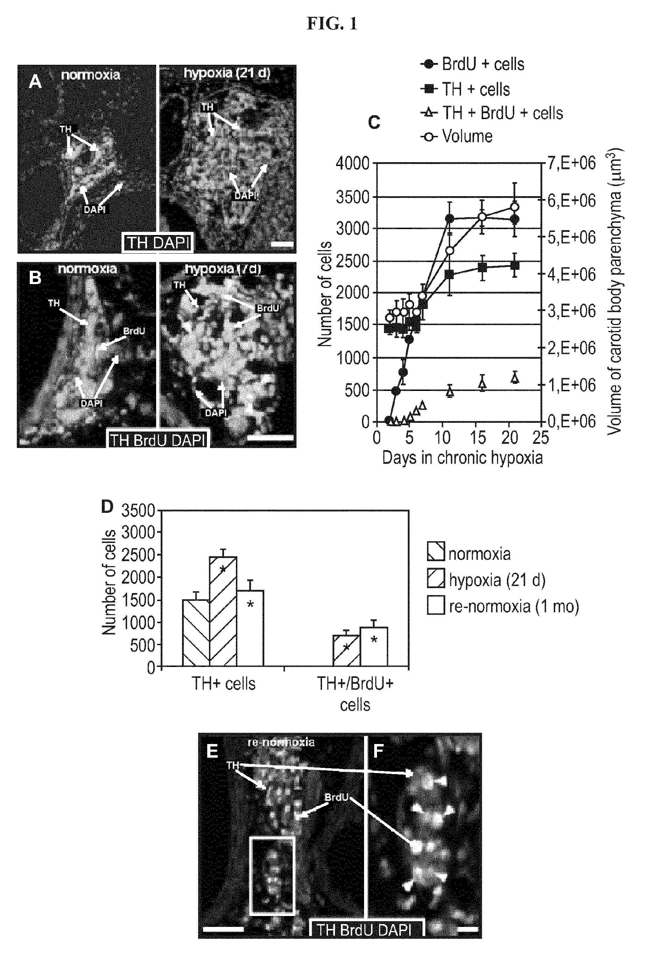

Fig. 1: Neurogenesis induced by chronic hypoxia in the adult carotid body. Immunocytochemical analysis of carotid bodies isolated from mice treated with BrdU and exposed to chronic hypoxia (10% O₂). Panel A shows the typical growth of the mass of neuronal TH+ glomus cells in response to the hypoxic stimulus (21 days). Panel B shows the incorporation of BrdU in the proliferating cells after 7 days of hypoxia, and the consequent marking of the differentiated progeny. TH staining indicates glomus cells, staining with BrdU indicates proliferating cells, and DAPI fluorescence shows the nuclei. Panel C: Variation in the number of cells and in the volume of the parenchyma of the carotid body (ordinate) as a function of time in animals exposed to chronic hypoxia (abscissa). Panel D: Reversibility of the growth of the carotid body in animals returned to normoxia (21% O₂) for 1 month. The bar chart compares a quantification of the number of TH+ cells and TH and BrdU cells double-positive in the renormoxic carotid bodies, with the values of normoxia and hypoxia (* significantly different in normoxia, p<0.01; # significantly different in hypoxia, p<0.05). Panels E and F show, at different magnification, an example of a section of renormoxic carotid body, showing that approximately half of the glomus cells have been replaced by new cells. Scale: 50 microns in panels A, B and E, and 10 microns in panel F.

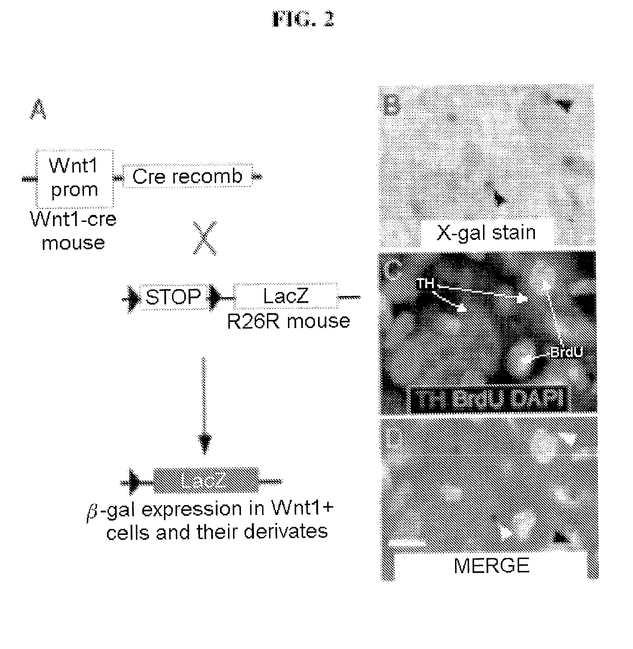

Fig. 2: Origin of the neural crest (NC) of the preexisting and recently formed TH+ glomus cells. Panel A: Strategy for investigation of cells derived from the neural crest in the carotid body using a loxP-flanked Wnt1-cre/LacZ transgenic mouse. In this animal model the cells that are positive for Wnt1, and all their derivatives, express galactosidase, and therefore show a blue precipitate after staining with X-gal (arrowheads in panel B). Panel C: Immunohistochemical detection of TH, BrdU, and of nuclei (DAPI), in the same section shown in B. Panel D shows the two images combined, showing that the glomus cells, both BrdU positive and BrdU negative, derive from the neural crest. The BrdU+, TH- and LacZ+ cell marked by the black arrowhead might represent a transient amplifying progenitor. Scale: 10 microns.

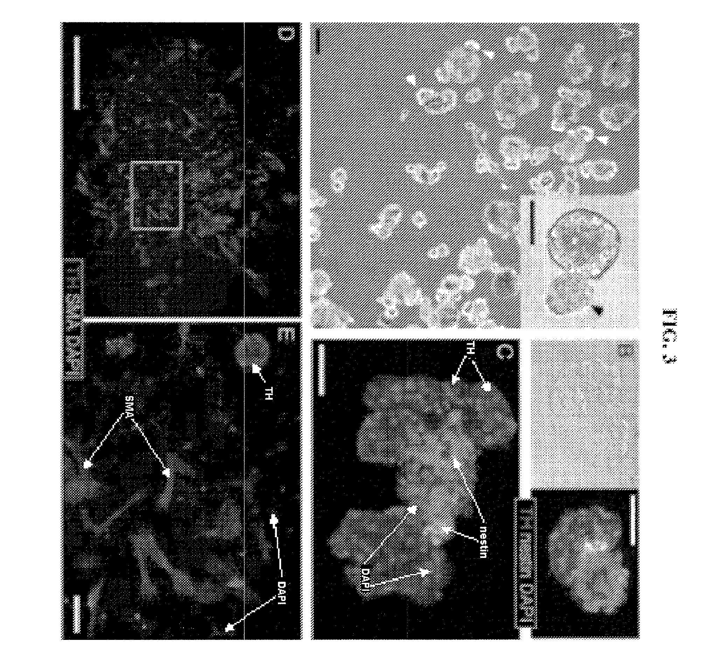

Fig. 3: Formation of neurospheres and pluripotency of the stem cells from the carotid body *in vitro.* Panel A: Light-field photograph of the neurospheres resulting after culture for 10 days. The detail shows an example of the typical thickenings or glomeruli (arrowheads) that appear in the neurospheres. Panel B shows an immunohistochemical analysis of a thin section of neurosphere (light field on the left), showing the presence of nestin+ progenitors in the nucleus of the neurosphere, and of TH+ glomus cells in the glomerulus (right). Panel C shows a neurosphere that has grown considerably (20 days in culture) with two long glomeruli containing numerous differentiated TH+ cells. Panel D shows the differentiation to smooth muscle cell (SMA+) of the carotid body progenitors after replating the neurospheres on adherent substrate. Panel E shows an image of the area in the box in D, at higher magnification. Note the presence of TH+ glomus cells at the centre of the colony. Scale: 100 microns in panels A and E, 50 microns in panels B and C, and 1 mm in panel D.

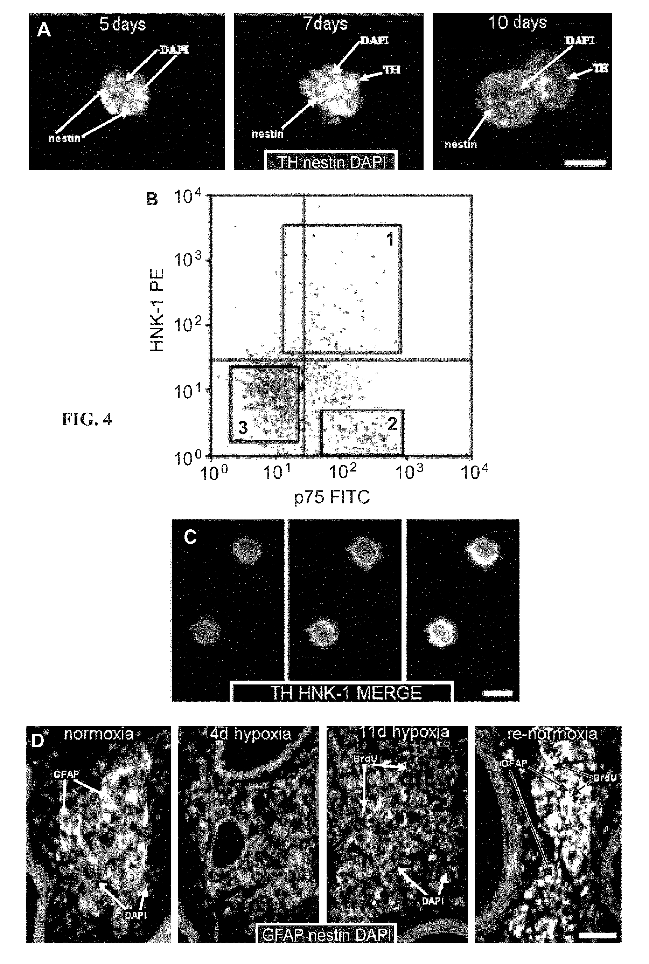

Fig. 4: Identification of the stem cells from the carotid body.Panel A: Formation *in vitro* of neurospheres of rat carotid body as a function of time. The appearance of glomus cells (TH+) is preceded by the formation of a nucleus of the neurosphere constituted of nestin+ progenitors. Panel B shows a flow cytometry analysis of the cells of the carotid body, using HNK-1 and p75 as markers. Only the double negative cells (selection window #3) were capable of growing, forming neurospheres in culture. The separation of HNK-1+ cells (window #1) and p75+ (window #2) always gave negative results. Panel C shows that the HNK-1+ cells were all TH+ glomus cells. In panel D we can see the immunohistochemical detection of the glial GFAP marker, and of BrdU, in the carotid bodies of normoxic, hypoxic, and renormoxic mice. The disappearance and subsequent appearance of staining with GFAP would suggest that the type II glial cells, activated by hypoxia, are the progenitor cells of the carotid body. Scale: 50 microns in panels A and D, and 10 microns in panel C.

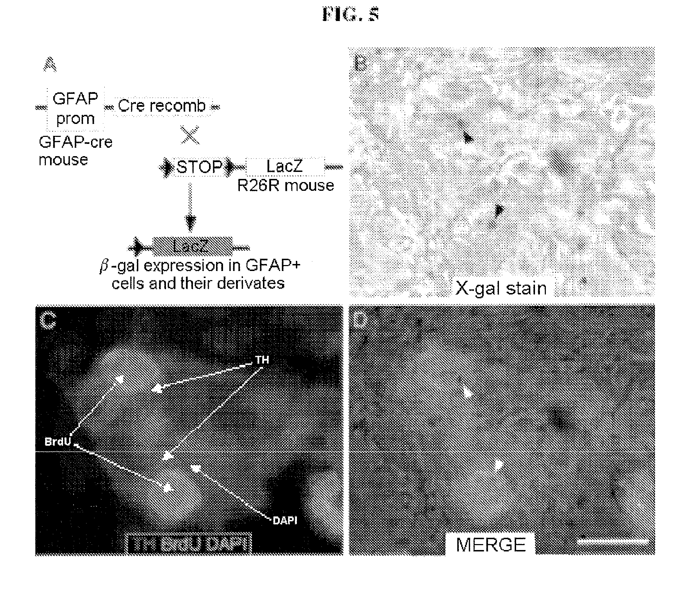

Fig. 5: The type II glial cells differentiate to type I neuronal cells in response to hypoxia. Panel A: Strategy for testing the glial lineage of the carotid body progenitors capable of differentiating to glomus cells. Immunohistochemical analysis of the carotid bodies of loxP-flanked GFAP-cre/LacZ transgenic mice subjected to hypoxia was used for investigating the derivatives from type II cells, which appeared as LacZ+. Panel B: X-gal staining of a thin section of carotid body, transgenic and hypoxic, indicating the presence of blue precipitate (arrowheads) in two different cells. Panel C shows the immunohistochemical detection of TH, BrdU and of the nuclei. The superposition (merge) of the two images in panel D shows that the two newly formed glomus cells in this glomerulus derive from type II GFAP+ cells. Scale: 10 microns.

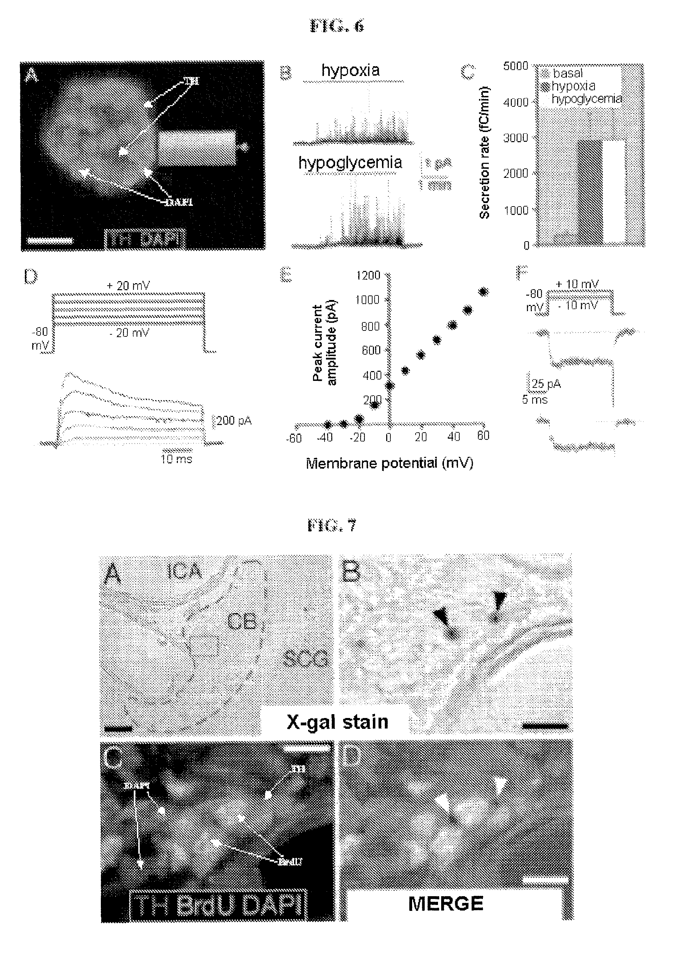

Fig. 6: The glomus cells generated from stem cells *in vitro* show mature chemoreceptor and electrophysiological responses. Panel A: Group of glomus cells (TH+) separated from a neurosphere, and diagram of a carbon fibre electrode used for recording the catecholaminergic secretion from a single cell. Panel B: Typical amperometric recordings obtained from groups of glomus cells in culture in response to physiological stimuli such as hypoxia (change in O₂ partial pressure from 145 mmHg to 15 mmHg) or hypoglycaemia (change from 5 mM glucose to 0 glucose). Each amperometric spike represents a single exocytotic event. Panel C: Quantitative summary of the rates of secretion induced by the drop in levels of O₂ and glucose. Panel D shows typical patch-clamp recordings with voltage-gated inflow (calcium) and outflow (potassium), recorded from a glomus cell present in a glomerulus of a neurosphere. Panel E shows the corresponding current/voltage curve for the potassium fluxes, and panel F shows patch-clamp recordings with calcium fluxes obtained after blocking the potassium channel with intracellular caesium. Note that these electrophysiological cellular properties, very similar to those described in mature glomus cells *in vivo,* are essential for the regulation of exocytosis in these chemoreceptor cells. Scale in panel A: 20 microns.
Fig. 7: The newly-formed glomus cells produce GDNF *in vivo.* Immunohistochemical analysis of the carotid bodies of heterozygotic GDNF/LacZ mice exposed to chronic hypoxia was carried out to observe the production of GDNF in newly-formed glomus cells. Panel A shows an image, at low magnification, of a thin section of one of these carotid bodies stained with X-gal. The positions of the superior cervical ganglion (SCG) and of the internal carotid artery (ICA) are also indicated. Note the concentration of the deposits of X-gal in the carotid body. Panel B shows an image at higher magnification, of the area in the box in A, with two light blue precipitates (arrowheads) demonstrating the expression of galactosidase and therefore the production of GDNF. Panel C: Immunohistochemical detection of TH, BrdU and the nuclei, in the same section. The superposition (merge) of images in panel D shows that the precipitates (arrowheads) correspond to production of GDNF in newly-formed glomus cells. Scale: 50 microns in panel A, and 10 microns in panels B, C and D.

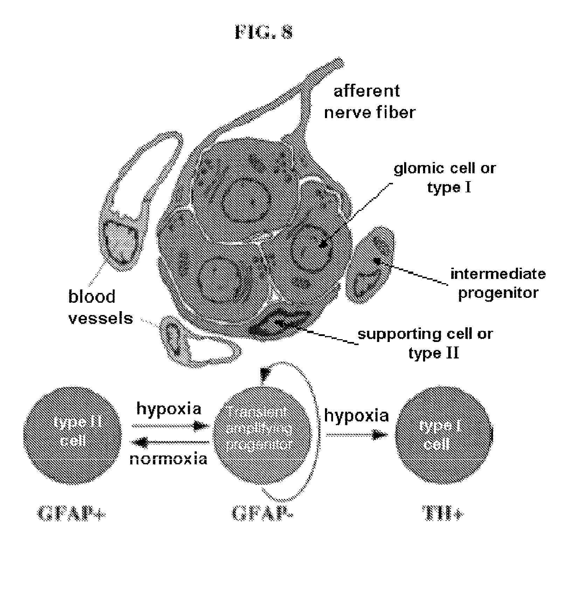

Fig. 8: Model of neurogenesis in the adult carotid body. Top. Diagram of a carotid body glomerulus with blood vessels and afferent nerve fibres encircling it. The glomus neuronal cells are surrounded by type II glial cells. A transient amplifying progenitor activated by hypoxia is also shown. Bottom. Hypothetical sequence of cellular events that occur in the carotid body during a cycle of hypoxia/renormoxia.

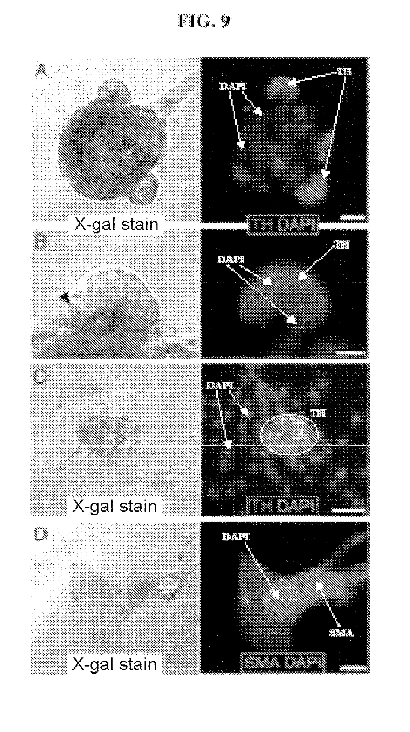

Fig. 9: Formation of neurospheres and pluripotency starting from GFAP+ stem cells of the carotid body. (A) left: Light-field photograph of a neurosphere arising from a GFAP+, X-gal+ cell dispersed from carotid bodies of loxP-flanked GFAP-cre/LacZ mice. Right: Immunocytochemical identification of TH+ cells (arrowheads). The enlarged images in (B) show the localization of the deposits of X-gal in TH+ cells (arrowheads). (C) left: Light-field photograph of a neurosphere from a GFAP+, X-gal+ cell cultivated on adherent substrate. Right: Immunocytochemical identification of TH+ cells (arrowheads). The enlarged images in (D) show the localization of the deposits of X-gal in SMA+ smooth muscle cells. Scale: 10 microns in panel (A), 5 microns in panels (B) and (D), and 50 microns in panel (C).

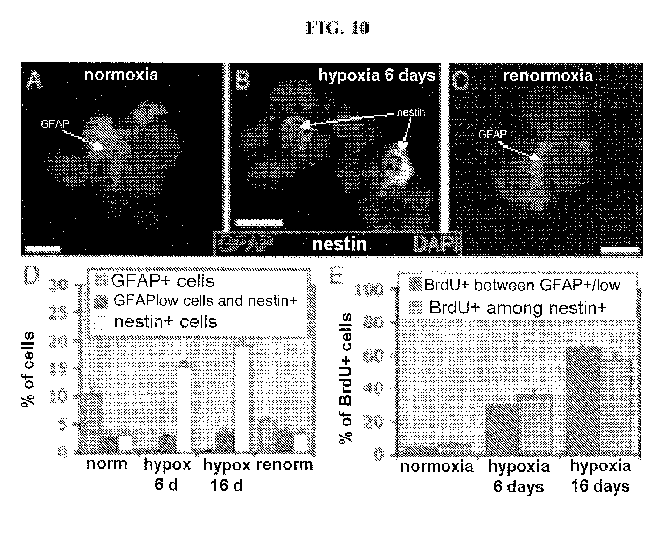

Fig. 10: Quantitative analysis of the progenitors of the carotid body during exposure to hypoxia/renormoxia *in vivo.* (A-C) Representative examples of GFAP+, nestin+ cells (arrowheads), and GFAP thin, nestin+, in carotid bodies of rat, normoxic or exposed to a cycle of hypoxia (16 days)/renormoxia (18 days). Scale: 5 microns in panels (A) and (C), and 10 microns in (B). (D) Quantitative chart showing the changes in the amount of the various cellular types of the carotid body during the experiment. For the counts of GFAP+ and/or nestin+ cells, the carotid bodies were dispersed and the cells were fixed for immunocytochemical analysis immediately after plating in culture. This procedure was necessary because the type II GFAP+ cells are difficult to identify in the tissue slices, and furthermore, staining with nestin also works better in dispersed cells. Note the marked decrease in GFAP+ cells in hypoxia, and the parallel increase in nestin+ cells. During renormoxia the reverse phenomenon occurs (recovery of the population of GFAP+ cells and decrease in the number of nestin+ cells). (E) Proliferation of GFAP+ or nestin+ progenitors of the carotid body during hypoxia. The mean values of the number of cells, calculated on the basis of three independent experiments, are: normoxia (3.6% GFAP+, BrdU+ of 111 GFAP+ cells; 5.7% nestin+, BrdU+ of 69 nestin+ cells), hypoxia 6 days (29.4% GFAP+, BrdU+ of 30 GFAP+ cells; 35.6% nestin+, BrdU+ of 247 nestin+ cells), hypoxia 16 days (64.3% GFAP+, BrdU+ of 25 GFAP+ cells; 57.8% nestin+, BrdU+ of 248 nestin+ cells).

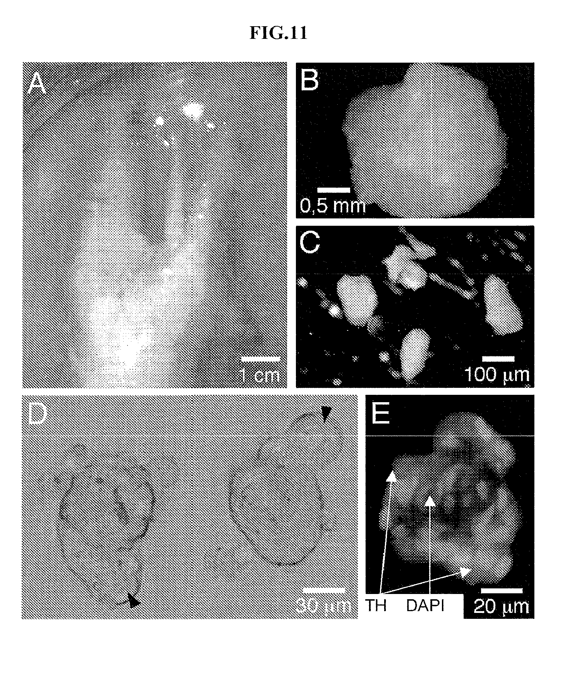

Fig. 11: Formation of neurospheres from human carotid body. Panel (A): Light-field photograph of a human carotid bifurcation prior to dissection of the carotid body. Panel (B) shows a human carotid body after dissection. Panel (C) shows, also in light field, various pieces of human carotid body prior to enzymatic treatment to produce cellular dispersion. Panel (D) shows a light-field photograph of two neurospheres obtained from dispersed cells of human carotid body after 10 days in culture. Note the presence of thickenings or glomeruli on the surface of the neurosphere (arrowheads), a morphological and functional characteristic very similar to that described in previous figures in rodents. Panel (E) shows, by immunocytochemical analysis against tyrosine hydroxylase (TH), that the aforesaid glomeruli are effectively glomus cells of the carotid body, differentiating from the progenitors in the neurosphere. The nuclei of all the cells have been stained with DAPI for greater clarity.

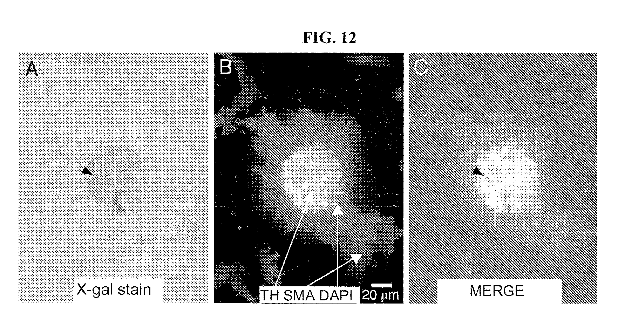

Fig. 12: The cells present in the neurospheres are able to produce GDNF *in vitro.* Panel A shows a light-field photograph of neurospheres obtained from a GDNF/LacZ mouse, resulting from culture for 10 days and stained with X-gal to verify expression of β-galactosidase controlled by the GDNF promoter in the cells of the neurospheres; the black arrow indicates the localization of blue deposits, indicating expression of the enzyme. Panel B presents the immunohistochemical analysis of a thin section of neurospheres, showing the presence of TH+ glomus cells in the glomerulus and their localization. The merging (superposition) of the two images shown in panel C shows how the appearance of the blue deposits of X-gal occurs in the localization of the TH+ glomus cells, demonstrating the possibility of obtaining expression *in vitro* of GDNF in these cells.

### DETAILED DESCRIPTION OF THE INVENTION

The present invention relates, in general, to adult stem cells obtained from the carotid body (CB) (stem cells of the invention), to progenitor cells derived from said adult stem cells (progenitor cells of the invention), to differentiated cells derived from said stem cells or progenitor cells of the invention (differentiated cells of the invention), to isolated cell populations that contain a set of said cells (cell population of the invention), to a method for their expansion (method of expansion of the invention), and to their applications, including their use in therapy, in particular in the treatment of neurodegenerative diseases (e.g. Alzheimer's disease, Parkinson's disease, etc.), or of diseases caused by ischaemic, traumatic or autoimmune lesions of the nervous system.

### Definitions

For better understanding of the present description, the meaning of some terms and expressions used in the context of this invention will be explained below. Additional definitions will be included along with the description when necessary.

The term "subject" as used here refers to an animal, preferably a mammal including a non-primate (e.g. cows, pigs, horses, cats, dogs, rats, mice, etc.) and a primate (e.g. monkeys, human beings, etc.). In a particular embodiment, said subject is a human being.

The term "adult stem cell" refers to a cell present in a differentiated tissue that displays characteristics of stem cells. "Adult" means post-embryonic. An adult stem cell is an undifferentiated cell that is in a differentiated tissue and displays capacity for proliferation, self-renewal and differentiation to one or more cellular types. The term means that the stem cell is isolated from a tissue or organ of an animal at a stage of growth later than the embryonic stage. The adult stem cells of the present invention are present in a subject's CB. The cells may be isolated preferably from a mammal, such as a human. Adult stem cells are unlike embryonic stem cells, which are defined by their origin, the inner cell mass of the blastocyst. Adult stem cells according to the invention may be isolated from any non-embryonic tissue, and will include neonates, juveniles, adolescents and adult patients. Generally the stem cell of the present invention will be isolated from a non-neonate mammal, and more preferably from a non-neonate human.

The term "derived progenitor cell" (also called transient amplifiers, transient amplifying cells, or intermediate progenitors in this description) denotes, in this description, a proliferating cell, arising from the stem cells of the invention, obtained from the CB, with capacity for differentiation to differentiated tissue cells, and more specialized than the stem cell. The term refers to a cell that has the capacity to create progeny that are more differentiated than itself. For example, the term may refer to an undifferentiated cell or a cell differentiated to an extent short of final differentiation which is capable of proliferation and giving rise to more progenitor cells having the ability to generate a large number of mother cells that can in turn give rise to differentiated, or differentiable daughter cells. A progenitor cell according to the invention is more differentiated than a true stem cell and has already somewhat restricted the multipotent capacity of the true stem cell. The term "derived differentiated or specialized cell" means, in this description, a cell that is terminally specialized for carrying out a defined function in the tissue, arising from the stem cells or derived progenitors, and that possesses little or no capacity for proliferation or for differentiation. The term "differentiation" refers to the formation of cells expressing markers known to be associated with cells that are more specialized and closer to becoming terminally differentiated cells that are incapable of further division or differentiation.

The term "reversibly immortalized cell" relates to a cell that, at a given moment, is in an immortalized state but that can be returned to a non-immortalized state at a later time, using a process of inverse immortalization.

The term "specialization or differentiation marker" means, in this description, a phenotypic marker that identifies the "differentiated or specialized cell" and distinguishes it from the stem cells and progenitor cells.

Cells of the invention are positive for certain phenotypic markers and negative for others. By "positive", it is meant that a marker is expressed within a cell. In order to be considered as being expressed, a marker must be present at a detectable level. By "detectable level" is meant that the marker can be detected using one of the standard laboratory methodologies such as PCR, blotting or FACS analysis. A gene is considered to be expressed by a cell of the population of the invention if expression can be reasonably detected after 30 PCR cycles, which corresponds to an expression level in the cell of at least about 100 copies per cell. The terms "express" and "expression" have corresponding meanings. At an expression level below this threshold, a marker is considered not to be expressed and the cell is then described as negative for this marker. A marker is considered not to be expressed by a cell of the invention, if expression cannot be reasonably detected at a level of about 10-20 copies per cell. In between these levels of positive and negative, a cell may be weakly positive for a particular marker e.g. progenitor cells according to the present invention are weakly positive for GFAP (±) and positive (+) for nestin. The comparison between the expression level of a marker in a cell of the invention, and the expression level of the same marker in another cell, such as for example an embryonic stem cell, may preferably be conducted by comparing the two cell types that have been isolated from the same species. Preferably this species is a mammal, and more preferably this species is human. Such comparison may conveniently be conducted using a reverse transcriptase polymerase chain reaction (RT-PCR) experiment.

A population of stem cells is considered to express a marker if at least about 70% of the cells of the population show detectable expression of the marker. In other aspects, at least about 80%, at least about 90% or at least about 95% or at least about 97% or at least about 98% or more of the cells of the population show detectable expression of the marker. In certain aspects, at least about 99% or 100% of the cells of the population show detectable expression of the markers. Expression may be detected through the use of an RT-PCR experiment, through fluorescence activated cell sorting (FACS), or through immunocytochemsitry using specific antibodies. It should be appreciated that this list is provided by way of example only, and is not intended to be limiting.

A population of stem cells is considered to be negative for a particular marker if at least about 70% of the cells of an isolated stem cell population does not show detectable expression of the marker. In other embodiments, at least about 80%, at least about 90% or at least about 95% or at least about 97% or at least about 98% or at least about 99% or 100% of the cells of a population should not show any detectable expression of the marker. Again, lack of detectable expression may be proven through the use of an RT-PCR experiment , using FACS, or using immunocytochemistry.

The term "isolated" indicates that the cell or cell population to which it refers is not within its natural environment. The cell or cell population has been substantially separated from surrounding tissue. In some embodiments, the cell or cell population is substantially separated from surrounding tissue if the sample contains at least about 75%, 85%, 90%, 95% or more stem cells, progenitor cells, differentiated cells, or immortalized cells according to the invention. In other words, the sample is substantially separated from the surrounding tissue if the sample contains less than about 25%, in some embodiments less than about 15%, and in some embodiments less than about 5% of materials other than the cells of the invention. Such percentage values refer to percentage by weight. The term encompasses cells which have been removed from the organism from which they originated, and exist in culture. The term also encompasses cells which have been removed from the organism from which they originated, and subsequently re-inserted into an organism. The organism which contains the re-inserted cells may be the same organism from which the cells were removed, or it may be a different organism.

The term "specific culture medium for neural crest-derived cells" (also called "NC medium" in this description) means, in this description, a medium that contains serum and a series of trophic factors that promote growth of progenitors of the peripheral nervous system (PNS), derived from the neural crest (NC). Examples of suitable trophic factors are described herein.

The term "molecule with biological activity" means, in this description, a molecule that has a defined effect on the biology of a group of cells, e.g. affects their survival or their proliferation or differentiation behaviour.

The term "therapeutically effective amount of cells" means, in this description, the amount of cells necessary for obtaining a beneficial effect in the treatment of a particular disease by means of cellular therapy.

The term "mixed culture" means, in this description, a culture of various different cellular types present on the same plate, and that may or may not derive from one another.

The term "pure culture" means, in this description, a culture with just one type of cell present, or very (substantially) enriched in just one type of cell. In practice it is technically challenging to obtain 100% pure cultures, although very enriched cultures can be obtained. In a particular embodiment, cultures of more than 70%, 80%, 90%, 95% or greater purity of stem cells of the invention [GFAP+/TH-/nestin-] or of differentiated cells of the invention [TH+/nestin-] can be obtained by separation by flow cytometry using specific fluorescent markers.

The term "cell population of the invention"refers to a preparation of cells, which preparation may include, in addition to the cells, non-cellular components such as cell culture media, e.g. proteins, amino acids, nucleic acids, nucleotides, co-enzyme, antioxidants, metals and the like. Furthermore, the cellular composition can have components which do not affect the growth or viability of the cellular component, but which are used to provide the cells in a particular format, e.g., as polymeric matrix for encapsulation or as a pharmaceutical preparation.

The term "mapping of the cell lineage" means, in this description, the tracking of a cell lineage in the sense of marking the progenitor cell for subsequent tracking of the marking and investigation of the cells that are derived from said progenitor cell and are therefore marked.

"Neurodegenerative diseases" are pathologies that affect the nervous system (generally the central nervous system) of known or unknown cause and that involve progressive death of neurons by apoptosis or necrosis; illustrative, non-limiting examples of said neurodegenerative diseases include Parkinson's disease, Alzheimer's disease, amyotrophic lateral sclerosis, etc.

"Ischaemic lesions" of the nervous system are lesions produced by lack of blood flow to a part of the brain, due to obstruction of the artery supplying blood to said region.

"Traumatic lesions" of the nervous system are characterized by destruction of neurons or parts thereof (axons, for example), due for example to mechanical injury (e.g. traumatic medullary lesions resulting from traffic accidents).

"Immunological lesions" of the nervous system (e.g. multiple sclerosis, etc.) result from autoimmune reactions, after the aberrant stimulation of the immune system. As used herein, the term "hypoxia" is defined to mean a condition in which oxygen in one or more tissues of a mammal is lowered below physiologic levels, e.g., to a less than optimal level. An example of a hypoxic solution is one which has been bubbled with 5% CO₂ and 95% N₂ to reach an O₂ pressure of approximately 15 mmHg.

As used herein, the term "culture medium specific to the neural crest" means a culture medium that allows growth of cells of this type. The term "culture medium" or "medium" is recognized in the art, and refers generally to any substance or preparation used for the cultivation of living cells. Most basal culture media generally comprise of four basic chemical groups: amino acids, carbohydrates, inorganic salts, and vitamins. A basal medium generally serves as the basis for a more complex medium, to which supplements such as serum, buffers, growth factors, lipids, and the like are added. Examples of basal media include, but are not limited to, Eagles Basal Medium, Minimum Essential Medium, Dulbecco's Modified Eagle's Medium, Medium 199, Nutrient Mixtures Ham's F-10 and Ham's F-12, McCoy's 5A, Dulbecco's MEM/F-I 2, RPMI 1640, and Iscove's Modified Dulbecco's Medium (IMDM). An example of a culture medium specific to the neural crest is D-MEM:F-12 (Gibco BRL, Grand Island, NY), containing 15% of chick embryo extract (prepared as described in Stemple and Anderson, 1992; Cell 71, 973-985), 1% of N2 supplement (Gibco), 2% of B27 supplement (Gibco), 1% of penicillin/streptomycin (Cambrex, Verviers, Belgium), 20 ng/ml of human recombinant basic fibroblast growth factor (bFGF) (R&D Systems, Minneapolis, MN), 20 ng/ml of human recombinant insulin-like growth factor-I (IGF-I) (R&D Systems), and 20 ng/ml that contained basic fibroblast growth factor (bFGF), insulin-like growth factor-I (IGF-I) and human recombinant epidermal growth factor (EGF) (R&D Systems). In this medium, cells can be cultured in incubators (e.g. Thermo Electron Corp., Waltham, MA) with controlled CO₂ levels, at a fixed oxygen level of 3% and at 37°C.

Throughout the description and the claims the word "comprises" and its variants are not intended to exclude other technical characteristics, additives, components or steps. For a person skilled in the art, other objects, advantages and characteristics of the invention will become clear partly from the description and partly from the practical application of the present invention.

### Cells and cell populations of the invention

### Stem cells of the invention

In one aspect, the invention relates to an isolated adult stem cell, originating from the CB (stem cell of the invention), characterized in that it is positive for the phenotypic marker GFAP (glial fibrillary acidic protein) and negative for the phenotypic markers TH and nestin. Therefore said stem cell of the invention is obtained from the CB and possesses a GFAP+/TH-/nestin- phenotype.

The particular features that define these stem cells from the carotid body can be summarized by the following characteristics:
- As already mentioned, they are cells that are positive for the glial GFAP marker and negative for the markers TH and nestin.
- They occur *in vivo* with cellular prolongations (usually one or two) around the glomeruli of type I cells, intimately associated with the membranes of both cellular types.
- They lose their resting phenotype (expression of GFAP and prolongations) in response to a continued hypoxic stimulus.
- On losing their resting phenotype, they are converted to more-rounded intermediate progenitors, positive for nestin and negative for GFAP or TH.
- They are capable of differentiating to mature glomus cells of the carotid body, positive for TH, and capable of secreting, in response to physiological stimuli, catecholamines such as dopamine and trophic factors such as GDNF. Thus, by inducing their differentiation, they can be used as a source for the production of GDNF.
- They are capable of recovering in *vivo* the quiescent phenotype (expression of GFAP and prolongations) when the hypoxic stimulus disappears.
- They are capable of expansion, growing *in vitro,* forming neurospheres or colonies of nestin positive progenitors and TH positive glomus cells.
- They may integrate into nerve tissue.
- They may establish chemical or electrochemical contact with adjacent cells.
- They may not induce an immune response when injected into a patient if they have been obtained from the CB of the mentioned patient.
- Additionally to being positive for the glial GFAP marker and negative for the markers TH and nestin, they are specific due to their localization (CB) and other functional properties such as colocalization with CB glomus cells, responsiveness to hypoxia, capability of growing in vitro forming neurospheres or colonies of nestin positive progenitors and TH positive glomus cells, etc.

The GFAP marker is characteristic of the glial cells of the brain, of the cells of the peripheral glia and of cells "similar" to the glial cells localized in various organs (for example the type II cells of the carotid body). GFAP is a protein of the cytoskeleton whose precise function is unknown.

The marker nestin is characteristic of several different types of neural progenitors in the central nervous system, the peripheral nervous system and in "paraneural" organs, such as the CB. The function of nestin is not known for certain.

TH is a specific marker of several different subtypes of catecholaminergic cells. The function of TH is to act as a catalyst of the first reaction (hydroxylation of tyrosine) that converts the amino acid tyrosine to a neurotransmitter in the catecholamine group (dopamine, noradrenaline or adrenaline).

Initially, the stem cells of the invention were identified by a method such as that described in Example 2. This method forms one aspect of the present invention. Briefly, CBs from adult rats exposed to hypoxia were dispersed enzymatically and the cells were plated in NC medium, on culture plates treated for non-adhesion, thus inducing growth in conditions of flotation and the production of neurospheres. The neurospheres are colonies originating from a single cell (clonal growth) that has the characteristics of proliferation and self-renewal (they are stem cells). On dispersing the carotid body and placing its cells in culture, only the stem cells (a small population of the total cells) have the capacity to form neurospheres. Next, the various cells present in said neurospheres were analysed by a standard immunocytochemical protocol that comprised incubating thin sections of said neurospheres blocked beforehand with a blocking solution with the primary antibodies (anti-GFAP, anti-TH and anti-nestin), and subsequently with the corresponding secondary antibodies, and finally contrastaining with DAPI (stains the cell nuclei). The results obtained revealed the existence of cells derived from the CB of rats submitted to hypoxia that possess a GFAP+/TH-/nestin- phenotype. These cells were then analysed, observing their capacity for proliferation, self-renewal and differentiation (Example 2). Additional tests revealed that said stem cells from the CB were sustentacular cells of glial type (type II) (Example 3).

Next, the same method was applied to the carotid body isolated from human donors, to verify the existence of stem cells from the carotid body in this species. This method forms a further aspect of the present invention. As described in Example 6, application of the method to two carotid bifurcations from heart donors made it possible to obtain, in culture, neural progenitors in the form of neurospheres, which had thickenings on their surface in the manner of glomeruli. Immunocytochemical analysis of the neurospheres revealed that the thickenings were effectively groups of TH+ glomus cells, confirming the existence of stem cells specific to the carotid body in human beings and demonstrating their capacity for differentiation to glomus cells *in vitro.*

Based on the information provided by this description, the stem cells of the invention can be obtained by conventional methods known by any person skilled in the art from the CB of a subject. In a particular embodiment, said subject is a mammal, such as a rodent (e.g. a rat or a mouse) or a primate (e.g. a human being).

The phenotypic markers of the stem cells of the invention can be identified by any suitable technique, usually based on positive/negative selection. In a particular embodiment, antibodies can be used, preferably monoclonal antibodies, to said phenotypic markers whose presence or absence in the stem cells of the invention must be confirmed with the aim of characterizing the stem cells of the invention on the basis of their immunocytochemical profile, although other conventional techniques known by a person skilled in the art can also be used, for example, RT-PCR. Therefore in a particular embodiment, monoclonal antibodies to GFAP are used for analysing for the presence of said marker in the selected cells and monoclonal antibodies to TH and nestin for analysing for the presence of said markers in said selected cells. Those cells that possess a GFAP+/TH-/nestin- phenotype, originate from CB and have the capacity for proliferation, self-renewal and differentiation to other cell types, constitute the stem cells of the invention. Said monoclonal antibodies are commercially available or can be obtained by a person skilled in the art by conventional methods. The section relating to Materials and Methods describes one means of carrying out the immunocytochemical characterization of different types of cells.

The stem cells of the invention can be expanded *in vitro* since they possess the capacity for proliferation and self-renewal. Briefly, said stem cells of the invention, once isolated and characterized, can be maintained and can proliferate in *vitro* in a suitable culture medium. In a particular embodiment, the stem cells of the invention are grown on a non-adherent surface to permit the formation of neurospheres. Moreover, in a particular embodiment, the culture medium (defined previously as NC medium) includes at least one growth factor selected from fibroblast growth factor (FGF), including basic fibroblast growth factor (bFGF), an epidermal growth factor (EGF), an insulin-like growth factor-I (IGF-I) and combinations thereof, preferably FGF at a concentration between 10 and 40 ng/ml, preferably 20 ng/ml. Alternatively, the culture medium can contain, in addition to FGF, other growth factors such as EGF and/or IGF-I at a concentration (of each of them) between 10 and 40 ng/ml, preferably 20 ng/ml. Said growth factors (FGF, EGF and IGF-I) can be native or recombinant, or alternatively functional variants of said growth factors (FGF, EGF and IGF-I) that are well known by a person skilled in the art, can also be used. In another particular embodiment, the culture medium contains, as principal source of nutrients, chick embryo extract at a concentration of at least 5% by weight of the total of the culture medium, preferably between 5% and 20%, and even more preferably 15%. Moreover, advantageously, with the aim of preventing contamination, the culture medium contains antibiotics, e.g. penicillin and/or streptomycin, at typical concentrations from 0.5 to 2% by weight relative to the total of the culture medium.

If desired, the stem cells of the invention can be expanded clonally using a method that is suitable for cloning cell populations (Example 3). Alternatively, a population of stem cells of the invention can be collected and the cells can be plated on separate plates (or in the wells of a multiwell plate). In another alternative embodiment, said stem cells can be subcloned on a multiwell plate in a random relation to facilitate the operation of placing a single cell in each well (e.g. from approximately 0.1 to about 1 cell/well). Of course, the stem cells of the invention can be cloned at low density (e.g. in a Petri dish) and can be isolated from other cells using suitable devices (e.g. cloning rings). The clonal population can be expanded in a suitable culture medium, e.g. an NC medium.

This aspect of the invention thus provides a population of isolated stem cells, wherein the cell population essentially comprises only cells of the invention, i.e. the cell population is pure. In many aspects, the cell population comprises at least about 70% (in other aspects at least 75%, 80%, 85%, 90%, 91%, 92%, 93%, 94%, 95%, 96%, 97%, 98%, 99%, 99.5%, 99.9% or 100%) of the stem cells of the invention.

The isolated cells can be cultivated until their developmental phenotype can be evaluated. The stem cells of the invention have the capacity for differentiating to different cellular types, such as to the progenitor cells of the invention (nestin+), for example, cells slightly or weakly positive for GFAP and positive for nestin (GFAP±/nestin+) or cells negative for GFAP and positive for nestin (GFAP-/nestin+), as well as to the differentiated cells of the invention (nestin-/specialization marker+), for example, TH+ (glomus cells), SMA+ (smooth muscle cells), etc. (see, for example, Example 2). The capacity of the stem cells of the invention for differentiating to one or more cellular types can be tested by conventional methods known by a person skilled in the art.

If desired, the stem cell of the invention can be modified genetically by any conventional method including, by way of illustration but non-limiting, processes of transgenesis, deletions or insertions in their genome, etc. One possible method used for obtaining immortalized cells, both stable and conditional, is based on stable transfection of the stem cells of the invention by lipofection with the plasmids pEF321T or pRITA, respectively, as discussed later in relation to the immortalized cells of the invention.

It is anticipated that the stem cells of the invention either do not trigger an immune response upon injection of a cell population into a patient when they have been obtained from the recipient patient. When donors are used to obtain the stem cells, the immune response can be minimized by matching the haplotypes of the donors to those of the recipients. In certain aspects of the invention, the stem cell population is considered not to trigger an immune response if at least about 70% of the cells of the isolated stem cell population do not trigger an immune response. In some embodiments, at least about 80%, at least about 90% or at least about 95%, 99% or more of the cells of the isolated stem cell population do not trigger an immune response. Preferably the cells of the invention do not trigger an antibody mediated immune response and/or do not trigger a humoral immune response and/or do not trigger a mixed lymphocyte immune response.

It will be understood by one skilled in the art that the ability of the cells of the invention to trigger an immune response can be tested in a variety of ways. By way of illustration only, it is possible to establish whether the cells of the invention trigger an immune response by culturing the cells of the invention with T-cells from an individual different from the stem cell donor. In order for cells to be capable of inducing an immune response, the culturing of such cells will result in stimulation of the proliferation of the T-cells. Assays for testing this capability are well known to the skilled reader.

In one embodiment, the cells of the invention will be considered not to trigger an immune response if the level of expression of one or more of IL2, IFNλ, TNFβ, IL4, IL5, IL10, IL3, TNFα and TGFβ, induced following incubation of the isolated stem cells with T cells from an individual different from the stem cell donor, is less than about 50%, preferably less than about at 40%, about 30%, about 25%, about 20% or about 10% or less, of the level of expression of one or more of IL2, IFNλ, TNFβ, IL4, IL5, IL10, IL3, TNFα and TGFβ following incubation of an equivalent T cell population with terminally differentiated cells isolated from a non-matched individual.

### Progenitor cells of the invention

In another aspect, the invention relates to an isolated adult progenitor cell, derived from said stem cell of the invention, called "progenitor cell of the invention" hereinafter, characterized in that it is positive for the phenotypic marker nestin; that is, the progenitor cell of the invention originates from the stem cell of the invention, i.e. from the CB, and possesses a nestin+ phenotype. In a particular embodiment said progenitor cell of the invention is slightly or weakly positive for the phenotypic marker GFAP and positive for the phenotypic marker nestin [GFAP±/nestin+], whereas, in another particular embodiment, said progenitor cell of the invention is negative for the phenotypic marker GFAP and positive for the phenotypic marker nestin [GFAP-/nestin+]. GFAP-/nestin+ cells exist that are neural progenitors but with origin and properties (with respect to the type of cells to which they differentiate) different from those discovered in the CB, such as the GFAP-/nestin+ cells derived from brain and striatum, described in US patent application US 2003/0095956, which differ from the progenitor cells of the invention not only in origin (brain or striatum vs. CB) but in that, furthermore, they lack the property of differentiating to glomus cells (TH+ and sensitive to hypoxia and hypoglycaemia), a property that is exhibited by the progenitor cells of the invention.

Accordingly, the properties that characterize these cells can be summarized as follows:
- They are cells that are positive for the marker nestin, and, in general, negative for the markers GFAP and TH, although some of them can be weakly positive for the phenotypic GFAP marker, depending on the moment of their development.
- They are rounded, or have very thin prolongations, and they are disposed *in vivo* together with the glomeruli of type 1 cells.
- They are cells that proliferate, and therefore increase in number, in response to a continued hypoxic stimulus.
- They are derived from the GFAP positive stem cells of the carotid body.
- They are capable of differentiating to mature glomus cells of the carotid body, positive for TH, and capable of secreting, in response to physiological stimuli, catecholamines such as dopamine and trophic factors such as GDNF. Thus, by inducing their differentiation, they can be used as a source for the production of GDNF.
- They are cells capable of transforming to GFAP positive quiescent stem cells of the carotid body when the hypoxic stimulus disappears.
- They are cells capable of growing *in vitro* forming neurospheres or colonies of nestin positive progenitors and TH positive glomus cells.

As already mentioned, in a particular embodiment the progenitor cell of the invention is slightly or weakly positive for the phenotypic marker GFAP and positive for the phenotypic marker nestin, that is, it has a GFAP+/nestin+ phenotype; this appears to be due to the fact that, among other possible reasons, as the stem cell of the invention [GFAP+/nestin-] differentiates to the progenitor cell of the invention, it acquires the nestin+ phenotype and loses the GFAP phenotype, so that depending on the moment of their development, progenitor cells of the invention can be observed that have a phenotype that is slightly or weakly positive for GFAP.

The progenitor cells of the invention can be obtained by a method such as that described in Example 2. This method forms one aspect of the present invention. Briefly, CB of adult rats exposed to hypoxia were dispersed enzymatically and the cells were plated in NC medium, in culture plates treated for non-adhesion, to obtain neurospheres. Immunocytochemical analysis of thin sections of said neurospheres with anti-GFAP and anti-nestin antibodies and contrastaining with DAPI revealed the existence of cells with a GFAP+/nestin+ phenotype and cells with a GFAP-/nestin+ phenotype in the central core of said neurospheres obtained from stem cells of the CB of rats subjected to hypoxia (progenitor cells of the invention). These cells were then analysed, observing their capacity for proliferation, self-renewal and differentiation (Example 2).

Based on the information provided by this description, the progenitor cells of the invention can be obtained by conventional methods known by any person skilled in the art from the CB, or from stem cells of the CB, of a subject. In a particular embodiment, said subject is a mammal, such as a rodent (e.g. a rat or a mouse) or a primate (e.g. a human being).

The phenotypic markers of the progenitor cells of the invention can be identified by any suitable technique, such as already mentioned. In a particular embodiment, it is possible to use antibodies, preferably monoclonal antibodies, to said phenotypic markers whose presence or absence in the progenitor cells of the invention must be confirmed with the aim of characterizing the progenitor cells of the invention by means of their immunocytochemical profile, although other conventional techniques known by a person skilled in the art can also be used. Therefore in a particular embodiment, monoclonal antibodies to GFAP are used for analysing the presence (slight) or absence of said marker in the selected cells and monoclonal antibodies to nestin for analysing for the presence of said marker in said selected cells. Those cells that possess a GFAP+/nestin+ or GFAP-/nestin+ phenotype, such as from the CB, or from stem cells of the CB, and have the capacity for proliferation, self-renewal and differentiation to other cellular types, constitute the progenitor cells of the invention. Said monoclonal antibodies are commercially available or can be obtained by a person skilled in the art by conventional methods. The section relating to Materials and Methods describes a manner of carrying out the immunocytochemical characterization of different types of cells.

The progenitor cells of the invention can be expanded in *vitro* since they possess the capacity for proliferation and self-renewal. Briefly, said progenitor cells of the invention, once isolated and characterized, can be maintained and can proliferate *in vitro* in a suitable culture medium. In a particular embodiment, the progenitor cells of the invention are grown on a non-adherent surface to permit the formation of neurospheres. Moreover, in a particular embodiment, the culture medium (defined previously as NC medium) comprises, at least one growth factor selected from FGF, including bFGF, EGF, IGF-I and combinations thereof, preferably FGF, at a concentration between 10 and 40 ng/ml, preferably 20 ng/ml. Alternatively, the culture medium can contain in addition to FGF, EGF and/or IGF-I, at a concentration (each of them) between 10 and 40 ng/ml, preferably 20 ng/ml. Said growth factors (FGF, EGF and IGF-I) can be native or recombinant, or alternatively functional variants of said growth factors (FGF, EGF and IGF-I) that are well known by a person skilled in the art can also be used. In another particular embodiment, the culture medium contains, as principal source of nutrients, chick embryo extract at a concentration of at least 5% by weight of the total of the culture medium, preferably between 5% and 20%, and even more preferably 15%. Moreover, advantageously, with the aim of preventing contamination, the culture medium contains antibiotics, e.g. penicillin and/or streptomycin, in typical concentrations from 0.5 to 2% by weight relative to the total of the culture medium.

If desired, the progenitor cells of the invention can be expanded clonally using a method that is suitable for cloning cell populations. Briefly, the neurospheres obtained in the primary culture can be dissociated mechanically or enzymatically to a single cell, and these cells can be cultivated again in NC medium under non-adherent conditions, for their secondary expansion.

The progenitor cells of the invention have the capacity for differentiating to different cellular types, such as to the differentiated cells of the invention (nestin-/specialization marker+), e.g, nestin-/TH+ cells (glomus cells), nestin-/SMA+ cells (smooth muscle cells), etc. (see, for example, Example 2). The capacity of the stem cells of the invention to differentiate to one or more cellular types can be tested by conventional methods known by a person skilled in the art.

If desired, the progenitor cell of the invention can be modified genetically by any conventional method including, by way of illustration but non-limiting, processes of transgenesis, deletions or insertions in its genome, etc. One of the possible methods used for obtaining immortalized cells, both stable and conditional, is based on the stable transfection of the progenitor cells of the invention by lipofection with the plasmids pEF321T or pRITA, respectively, as discussed later in relation to the immortalized cells of the invention.

### Differentiated cells of the invention

In another aspect, the invention relates to an adult differentiated cell, derived from any of the aforesaid cell types (that is, from the stem cell of the invention or from the progenitor cell of the invention), called "differentiated cell of the invention" hereinafter, characterized in that it is negative for the phenotypic marker nestin and is positive for a specialization marker (MdeE); that is, the differentiated cell of the invention originates from the stem cell of the invention or from the progenitor cell of the invention, i.e., from the CB, and possesses a nestin-/MdeE+ phenotype.

Once again, the properties that characterize these cells can be outlined as follows:
- They are cells that are positive for the marker TH, and negative for the markers GFAP and nestin.
- They are rounded and they group together forming glomeruli.
- They arise by differentiation starting from stem cells (GFAP+) and via progenitor cells (nestin+) of the carotid body, in response to a continued hypoxic stimulus.
- They are terminally differentiated cells, which possess voltage-gated calcium and potassium channels, and secrete neurotransmitters of the dopamine type in response to physiological stimuli such as hypoxia or hypoglycaemia.
- They perform the main chemoreceptor function of the carotid body.
- They are able to produce and secrete trophic factors of the GDNF type. Thus, they can be used for the production of GDNF.
- They appear in vitro in the form of glomeruli on the surface of the neurospheres, by differentiation starting from nestin-positive progenitors.

The differentiated cells of the invention can be obtained by a method that comprises cultivating the stem cells of the invention and/or the progenitor cells of the invention under conditions that permit the differentiation of said cells to the desired differentiated cells. Based on the information provided by this description, the differentiated cells of the invention can be obtained by conventional methods known by any person skilled in the art from the CB, or from stem cells of the CB, or from progenitor cells derived from said stem cells of the CB, of a subject. In a particular embodiment, said subject is a mammal, such as a rodent (e.g. a rat or a mouse) or a primate (e.g. a human being). The phenotypic markers of the differentiated cells of the invention can be identified by any suitable technique such as has already been mentioned, for example, using monoclonal antibodies to said phenotypic markers whose presence or absence in the differentiated cells of the invention must be confirmed, although other conventional techniques known by a person skilled in the art can also be used.

In a particular embodiment, said MdeE is tyrosine hydroxylase (TH) and said differentiated cell of the invention possesses a nestin-/TH+ phenotype (glomus cell). Briefly, said differentiated cells (glomusnestin-/TH+ cells) can be obtained by a method such as that described in Example 2. Briefly, CBs of adult rats exposed to hypoxia were dispersed enzymatically and the cells were plated in NC medium, on culture plates treated for non-adhesion, thereby obtaining neurospheres which, in contrast to the neurospheres derived from the CNS and from the PNS (generally spherical) had one or two protrusions of a characteristic form (Fig. 3A). Immunocytoehemical analysis of thin sections of said neurospheres with anti-nestin and anti-TH antibodies and contrastaining with DAPI revealed the existence of aggregates of differentiated cells, with a nestin-/TH+ phenotype, in the glomerular protrusions, joined to the central core via a narrow filament of tissue, which, after several weeks in culture, grow to reach the size of an adult CB (Fig. 3C).

In another particular embodiment, said MdeE is smooth muscle actin (SMA) and said differentiated cell of the invention possesses a nestin-/SMA+ phenotype (a cell type typically derived from the neural crest). Briefly, said differentiated cells (nestin-/SMA+) can be obtained by a method as described in Example 2. Briefly, neurospheres (obtained from the CB of adult rats exposed to hypoxia) are re-plated in culture on an adherent substrate. Immunocytochemical analysis of the resultant cells with anti-nestin and anti-SMA antibodies and contrastaining with DAPI revealed the existence of differentiated cells, with a nestin-/SMA+ phenotype (Fig. 3D).

If desired, the differentiated cell of the invention can be modified genetically by any conventional method including, by way of illustration but non-limiting, processes of transgenesis, deletions or insertions in its genome, etc. One of the possible methods used for obtaining immortalized cells, both stable and conditional, is based on the stable transfection of the progenitor cells of the invention by lipofection with the plasmids pEF321T or pRITA, respectively, as discussed later in relation to the immortalized cells of the invention.

### Immortalized cells of the invention

The stem cells of the invention, the progenitor cells of the invention and/or the differentiated cells of the invention, called "cells of the invention" hereinafter, can if desired undergo a process of reversible immortalization.

Accordingly, in another aspect, the invention relates to a reversibly immortalized cell, called an "immortalized cell of the invention" hereinafter, said cell being a cell of the invention, that is, a stem cell of the invention, a progenitor cell of the invention or a differentiated cell of the invention.

In a particular embodiment, the cells of the invention undergo a process of reversible immortalization with the aim of obtaining functional, reversibly immortalized cells of the invention that can be used for therapeutic purposes, e.g. to promote the regeneration of nerve cells by implanting said cells, or preferably, differentiated cells derived from said stem cells, in those regions of the CNS or of the PNS where the damage has occurred.

As used here, the term "immortalization" or "immortalized" relates to a cell, or to a process for the creation of a cell, that will proliferate indefinitely in culture, avoiding ageing. According to the present invention, immortalization relates to a process by which a cell culture is transformed in such a way that the cells behave in some respects like tumour cells, in particular with respect to the proliferative characteristic of tumour cells. Thus, a "reversibly immortalized cell" relates to a cell which, at a given moment, is in an immortalized state, but which can be returned to a non-immortalized state at a later time, using a process of inverse immortalization.

In a particular embodiment, said cells of the invention can be reversibly immortalized by a method that comprises immortalizing cells of the invention by transformation with a vector that includes a "removable polynucleotide" that contains an oncogene (or a combination of oncogenes) to prevent ageing, such as the gene that encodes the long arm of the T antigen of the SV40 virus, the gene that encodes the catalytic subunit of telomerase, the gene that encodes the Bmi-1 protein, etc., thereby producing immortalized cells of the invention; growing said immortalized cells of the invention; selecting the clonal cell lines of cells of the invention that maintain the functional properties of the cells of the invention, and removing the oncogene (or combination of oncogenes) from the immortalized cells of the invention.

By way of illustration, the methods that can potentially be used for obtaining immortalized cells of the invention, both stable and conditional, include methods based on the stable transfection of the dispersed cells from primary culture of CB, by lipofection with the plasmids pEF321T or pRITA, respectively.

The plasmid pEF321T [Kim et al., 1990, Gene 91, 217-223] expresses the long arm of the T antigen of the SV40 virus, under the ubiquitous promoter of the elongation factor 1α (EF-1α), which is capable of immortalizing the cells that express it. This antigen activates a series of proto-oncogenes that transform the cell into a tumoral cell. Then, using techniques of molecular biology, histology and immunohistochemistry, the various clones obtained will be characterized, with the aim of identifying which cell type, among those making up the CB, has been immortalized in each of them.

The plasmid pRITA (Reversible Immortalization with T antigen) [Tobias M. et al., 2004, Nucleic Acids Research, 32, 5529-5538] possesses a bidirectional promoter P_{biTA.} to which the transactivatortTAthat responds to doxycycline is bound. This activator permits expression of the mRNA of the T antigen of SV40, and hence cellular proliferation, in the presence of doxycycline, transforming the cells to reversibly immortal. In the absence of doxycycline, the mRNA of the repressor rtTA2M2 (repressor of the T antigen of SV40) is expressed, making the immortalization reversible, and hence slowing down cellular proliferation. Expression of the repressor, in its turn, is linked to that of the gene that encodes the green fluorescent protein (GFP) and to that of a neomycin resistance cassette, which make it possible to select the clones with neomycin and verify the reversibility of the immortalization with a fluorescence microscope. Various clones are selected before the proliferation reverts and they are characterized by techniques of molecular biology, immunohistochemistry and electrophysiology. The clones with GFAP+ cells (stem cells of the invention) can be identified by PCR and selected. Moreover, if desired, in those clones that possess typical markers of glomus cells (TH+) and that produce GDNF, reversion of the proliferation process is possible, by removing the doxycycline from the medium and adding neomycin, and, if desired, they are used in intrastriatal implants of animal models of Parkinson's disease.

In a particular embodiment, said reversibly immortalized cells of the invention are reversibly immortalized stem cells of the invention, or reversibly immortalized progenitor cells of the invention, or reversibly immortalized differentiated cells of the invention, and advantageously, reversibly immortalized differentiated cells of the invention.

In another particular embodiment, the invention provides a combination of two or more types of reversibly immortalized cells of the invention, that is, of reversibly immortalized stem cells of the invention and/or reversibly immortalized progenitor cells of the invention and/or reversibly immortalized differentiated cells of the invention.

### Cell population of the invention

In another aspect, the invention relates to an isolated population of cells derived from CB, called "cell population of the invention" hereinafter, that comprises a set of cells selected from the group comprising the stem cells of the invention, the progenitor cells of the invention, the differentiated cells of the invention, the immortalized cells of the invention, and combinations thereof. The characteristics of said cells have already been mentioned. The cells of the invention can be stored for a long period in a suitable medium, e.g. NC medium, with high serum content (typically greater than 20%) and a cryopreservative, such as dimethylsulphoxide (DMSO), in an amount of approximately 10% by weight, by freezing at -80°C.

Said cell population of the invention can, if desired, be in a cell bank for transplant. In a particular embodiment, said cell bank comprises a plurality of reversibly immortalized cells of the invention, preferably of reversibly immortalized differentiated cells of the invention, homozygotic for at least one or more genes of critical antigens, that is, genes that encode histocompatibility antigens (e.g. an allele of the major histocompatibility complex (MHC) present in the human population). From said bank, it is possible to select the reversibly immortalized cells of the invention that are homozygotic for one or more alleles of histocompatibility antigens compatible with the allele of the MHC of a subject needing a transplant.

If desired, the cell population of the invention can be kept frozen under conditions that neither affect nor compromise its viability after reconstitution.

### Expansion of stem cells, progenitor cells, and differentiated cells of the invention

In another aspect, the invention relates to a method for the expansion or preparation of said stem cells of the invention or of said progenitor cells of the invention,called "method of expansion of the invention" hereinafter, that comprises cultivating said stem cells or progenitor cells of the invention under conditions of hypoxia in a culture medium that is specific for the neural crest.

According to this aspect of the invention, therefore, there is provided a method of preparing a stem cell according to any one of the aspects of the invention described above, the method comprising dispersing a carotid body isolated from an organism exposed to hypoxia, and isolating those cells which have the capacity to form a neurosphere. Preferably, cells of the invention are isolated from glomus tissue. Suitable cells are sustentacular cells of glial type (type II).

The glomus tissue is preferably dispersed enzymatically, using techniques known in the art. Enzymes suitable for dispersion include collagenase(types I, II and/or IV), trypsin and elastase. The glomus tissue can be further dispersed, for example, using fine tweezers, and/or by pipetting them mechanically.

In a particular embodiment, said stem cells or progenitor cells of the invention are cultivated on a non-adherent surface to permit the formation of neurospheres, which allows culturing under the conditions of flotation necessary for neurosphere formation.

Said culture medium that is specific for the neural crest comprises a culture medium, a source of nutrients, antibiotics and a growth factor. In a particular embodiment, said culture medium is DMEM:F-12; said source of nutrients comprises a chick embryo extract, preferably embryonic, at a concentration of at least 5% of the total of the culture medium, preferably between 5% and 20%, more preferably 15%; said antibiotics are selected from penicillin, streptomycin and mixtures thereof, in typical concentrations from 0.5 to 2% by weight relative to the total of the culture medium; and said growth factor is selected from FGF, including bFGF, or a functional variant thereof, IGF-I or a functional variant thereof, EGF or a variant thereof, and a combination of said factors, at a concentration (each of them) between 10 and 40 ng/ml, preferably, 20 ng/ml. Said growth factors (FGF, EGF and IGF-I) can be native or recombinant, although, as an alternative, it is also possible to use functional variants of said growth factors that are well known by a person skilled in the art. In a concrete embodiment, said culture medium that is specific for the neural crest (NC medium) comprises human recombinant bFGF, human recombinant IGF-I and human recombinant EGF. In addition, if necessary, the culture medium that is specific for the neural crest can be supplemented with a source of nitrogen and with B27.

Culture is carried out in conditions of hypoxia, that is, in the presence of an oxygen concentration of less than 15%, preferably between 1-10%, more preferably between 2-5% and even more preferably 3% (see Morrison et al., 2000, J Neurosci 20, 7370-7376). Such conditions stimulate clonogenic proliferation.

Moreover, said culture is carried out at a temperature between 35°C and 39°C, preferably at 37°C.

With the method of expansion of the invention it is possible to obtain a substantial amount, such as a therapeutically effective amount, either of pure culture of any of said cellular types mentioned (stem cells of the invention or progenitor cells of the invention) or of mixed culture of said cellular types.

The cells produced according to these methods may be detected by observing their capacity for proliferation, self-renewal and differentiation. For testing the capacity for self-renewal, individual primary neurospherescan be re-plated on adherent substrate treated with poly-L-lysine, and incubated for 48 hours with NC medium. Next, they are detached with trypsin and dispersed mechanically, finally being cultivated again on non-adherent plates with NC medium. Secondary neurospherescan be counted after 10 days of culture.

The cells may be detected using one, two or three antibodies that each bind to one of GFAP, TH and nestin. Suitable antibodies aer commercially available e.g. anti-TH antibody produced in rabbit (1:1000, Pel-Freez, Roger, AR), and anti-GFAP antibody also produced in rabbit (1:500, DAKO Cytomation, Fort Collins, CO).To detect a marker by immunohistochemical analysis, tissue sections can be used. First, the sections are pre-blocked for 1 hour at room temperature with blocking solution (phosphate buffer plus 10% of goat serum, 0.1% of bovine serum albumin (BSA), and 0.3% of Triton X-100 (Sigma)). The primary antibody to the marker (diluted e.g. 1:100) is incubated on the tissue overnight at 4°C, followed by incubation for 4 hours at room temperature with a secondary antibody (e.g. fluorescein-conjugated secondary antibody diluted, e.g. 1:200; Jackson Immunoresearch, West Grove, PA; or secondary antibody conjugated with Alexa 568 (1:500, Molecular Probes, Eugene, OR). Finally, the tissues may be stained with e.g. 2.5 mg/ml of 4',6-diamidino-2-phenylindole dihydrochloride [DAPI] (Sigma) for 10 minutes at room temperature for marking the nuclei, prior to assembly with 70% of glycerol prepared in phosphate-buffered saline [PBS].

The particular method used for isolation of the cells is not an essential feature of the invention. Nevertheless, with the knowledge of the physical and functional features of the stem cell of the invention, a number of methods are known in the art which can be utilised to isolate these cells from tissue. This method is provided by way of illustration only, and is not intended to be limiting.

Of course, any one of a number of physical methods of separation known in the art, alternative to those described above, may be used to select the cells of the invention and distinguish these from other cell types. Such physical methods may involve FACS and various conventionalimmuno-affinity methods based upon makers specifically expressed by the cells of the invention. For example, it will be apparent to a person skilled in the art that FACS analysis using an anti-GFAP antibody will provide a purified cell population. However, in some embodiments, it may be preferable to purify the cell population further by performing a further round of FACS analysis using one or more of the other identifiable markers listed above, optionally in conjunction with known stem cell markers, either by positive or negative selection.

### Applications

### Pharmaceutical composition

In another aspect, the invention relates to a pharmaceutical composition, hereinafter pharmaceutical composition of the invention, that includes a therapeutically effective amount of said stem cells, progenitor cells or differentiated cells of the invention, or combinations thereof, or of said immortalized cells of the invention, or of said cell population of the invention, and a pharmaceutically acceptable excipient.

The intrinsic characteristics of said cells and cell populations, including those of the reversibly immortalized cells, have been described previously.

In a particular embodiment, the pharmaceutical composition of the invention comprises a therapeutically effective amount of said differentiated cells of the invention, or of said reversibly immortalized differentiated cells of the invention, or of a cell population of the invention that comprises said differentiated cells of the invention and/or said reversibly immortalized differentiated cells of the invention.

As used here, the term "therapeutically effective amount" relates to the amount of stem cells, progenitor cells or differentiated cells of the invention, or of combinations thereof, or of said immortalized cells of the invention, or of said cell population of the invention, that the pharmaceutical composition of the invention that is capable of producing the desired therapeutic effect, must contain; in general, said therapeutically effective amount will be determined by, among other factors, the intrinsic characteristics of the cells and the desired therapeutic effect that is pursued. In general, the therapeutically effective amount of cells of the invention that must be administered will depend, among other factors, on the severity of the disease to be treated, on the intrinsic characteristics of the subject, on the region affected, etc. For this reason, the doses mentioned in this description must only be regarded as a guide for an expert in this area, who must adjust said dose depending on the factors described above. By way of illustration but non-limiting, the pharmaceutical composition of the invention can be administered as a single dose, that contains approximately between 1x10⁵ and 1x10⁹, preferably between 1x10⁶ and 1x10⁸, more preferably between 1x10⁷ and 5x10⁷ stem cells, progenitor cells or differentiated cells of the invention, or combinations thereof, or of immortalized cells of the invention. The dose can be repeated, depending on the patient's condition and progress, at time intervals of days, weeks or months, which have to be established by the specialist in each case.

The stem cells, progenitor cells or differentiated cells of the invention, the immortalized cells of the invention, as well as the cells present in the cell population of the invention, can be cells of autologous, allogeneic or xenogeneic origin. In a particular embodiment, said cells are of autologous origin and are isolated from the CB of the subject to whom they will be administered, thus reducing potential complications associated with antigenic and/or immunogenic responses to said cells.

If desired, the stem cells, progenitor cells or differentiated cells of the invention, or combinations thereof, the immortalized cells of the invention as well as those of the cell population of the invention can be purified, as mentioned previously, using selection of positive and/or negative cells by means of antibodies with the aim of selecting a single type of cell or of enriching the cell population to increase the efficacy, reduce the morbidity or facilitate the method. In a particular embodiment, said pharmaceutical composition of the invention comprises mostly differentiated cells of the invention, e.g. glomus cells (TH+/nestin-), or reversibly immortalized differentiated cells of the invention, e.g. reversibly immortalized glomus cells, or a cell population of the invention substantially enriched in said cells, e.g. glomus cells.

In accordance with the invention, the stem cells, progenitor cells or differentiated cells of the invention, the reversibly immortalized cells of the invention, as well as the cells of the cell population of the invention, can be administered to the subject without the need to apply additional processing to them or after applying additional processes for purifying said cells. Moreover, said stem cells, progenitor cells or differentiated cells of the invention, said immortalized cells of the invention, as well as the cells of the cell population of the invention, can be administered either separately, or together with other cell populations, e.g. together with other cells of the region of the CNS or of the PNS where they will be implanted.

The term "pharmaceutically acceptable excipient" as used here refers to the fact that it must be approved by a regulatory agency of the federal government or a national government or one listed in the United States Pharmacopoeia or the European Pharmacopoeia, or some other pharmacopoeia generally recognized for use in animals and in humans. The term "vehicle" relates to a diluent, excipient, carrier or adjuvant with which the stem cells, progenitor cells or differentiated cells of the invention, the immortalized cells of the invention, as well as the cells of the cell population of the invention, must be administered; obviously, said vehicle must be compatible with the cells. Illustrative, non-limiting examples of said vehicle include any physiologically compatible vehicle, for example isotonic solutions (e.g. sterile saline solution (0.9% NaCl), phosphate-buffered saline solution (PBS), Ringer-lactate solution, etc.), optionally supplemented with serum, preferably with autologous serum; culture media (e.g. DMEM, RPMI, McCoy, etc.); or, preferably, a solid, semisolid, gelatinous or viscous support medium, such as collagen, collagen-glycosamine-glycan, fibrin, polyvinyl chloride, poly-amino acids, such as polylysine, or polyornithine, hydrogels, agarose, dextran sulphate silicone. Moreover, if desired, the support medium can, in special embodiments, contain growth factors or other agents. If the support is solid, semisolid, or gelatinous, the cells can be introduced in a liquid phase of the vehicle that is treated subsequently so that it is converted into a more solid phase. In some embodiments of the invention in which the vehicle has a solid structure, said vehicle can be configured according to the form of the lesion.

The pharmaceutical composition of the invention can, if desired, also contain, when necessary, additives for increasing and/or controlling the desired therapeutic effect of the cells, e.g. buffering agents, surface-active agents, preservatives, etc. The pharmaceutically acceptable carrier may comprise a cell culture medium which supports the cells' viability. The medium will generally be serum-free in order to avoid provoking an immune response in the recipient. The carrier will generally be buffered and/or pyrogen free. Also, for stabilizing the cellular suspension, it is possible to add chelating agents of metals. The stability of the cells in the liquid medium of the pharmaceutical composition of the invention can be improved by adding additional substances, such as, for example, aspartic acid, glutamic acid, etc. Said pharmaceutically acceptable substances that can be used in the pharmaceutical composition of the invention are generally known by a person skilled in the art and are normally used in the production of cellular compositions. Examples of suitable pharmaceutical vehicles are described in "Remington's Pharmaceutical Sciences" by E.W. Martin. Additional information on said vehicles can be found in any manual of pharmaceutical technology (that is, galenical pharmacy).

The pharmaceutical composition of the invention will be administered in a suitable pharmaceutical form of administration. For this, the pharmaceutical composition of the invention will be formulated according to the chosen form of administration. The formulation will be adapted to the method of administration. In a special embodiment, the pharmaceutical composition is prepared in a liquid, solid or semisolid dosage form, e.g. in the form of suspension, in order to be administered by implanting, injection or infusion to the subject needing treatment. Illustrative, non-limiting examples include formulation of the pharmaceutical composition of the invention in a sterile suspension with a pharmaceutically acceptable excipient, e.g. an isotonic solution, for example, phosphate-buffered saline solution (PBS), or any other suitable, pharmaceutically acceptable vehicle, for administration to a subject parenterally, although other routes of administration can also be used.

The administration of the pharmaceutical composition of the invention to the subject who needs it will be carried out using conventional means. In a particular embodiment, said pharmaceutical composition of the invention can be administered to the subject parenterally using suitable devices such as syringes, catheters, trocars, cannulas, etc. In all cases, the pharmaceutical composition of the invention will be administered using equipment, apparatus and devices suitable for the administration of cellular compositions and known by a person skilled in the art.

In another embodiment, direct administration of the pharmaceutical composition of the invention to the site that is intended to benefit may be advantageous. In this method, direct administration of the pharmaceutical composition of the invention to the desired organ or tissue can be achieved by direct administration (e.g. by injection, etc.) on the external surface of the affected organ or tissue by inserting a suitable device, e.g. a suitable cannula, by infusion (including reverse flow mechanisms) or by other means described in this patent or known in the art. In a preferred embodiment, the pharmaceutical composition of the invention will be administered directly into the damaged region of the CNS or of the PNS.

The pharmaceutical composition of the invention can be stored until the moment of its application by the conventional methods known by a person skilled in the art. For short-term storage (less than 6 hours), the pharmaceutical composition of the invention can be stored at or below room temperature in a sealed container, supplemented or not with a nutrient solution. Medium-term storage (less than 48 hours) is preferably carried out at 2-8°C, and the pharmaceutical composition of the invention includes, in addition, an iso-osmotic, buffered solution in a container made of or lined with a material that prevents cellular adhesion. Longer-term storage is preferably carried out by means of suitable cryopreservation and storage in conditions that promote the retention of cellular function.

In a concrete embodiment, the pharmaceutical composition of the invention can be used in combination therapy. In a preferred concrete embodiment, said pharmaceutical composition is administered in combination with an additional pharmaceutical composition for the treatment of neurodegenerative diseases or for the treatment of diseases caused by ischaemic, traumatic or autoimmune lesions of the nervous system. Thus, the cells of the invention can be used as monotherapy or as therapy combined with other conventional methods for the treatment of said diseases.

The pharmaceutical composition of the invention can be used in a combination therapy with additional medication useful in the treatment of neurodegenerative diseases or for the treatment of diseases caused by ischaemic, traumatic or autoimmune lesions of the nervous system, as has already been described. Said additional medicinal products can form part of the same pharmaceutical composition or can, alternatively, be supplied in the form of a separate composition for simultaneous or successive (sequential in time) administration relative to the administration of the pharmaceutical composition of the invention. In a concrete embodiment, said additional pharmaceutical composition is administered simultaneously or sequentially to the pharmaceutical composition that contains the stem cells, progenitor cells and/or differentiated cells of the invention, or the cell population of the invention, spread out over time, in any order, i.e. the pharmaceutical composition of the invention can be administered first, then the other additional medicinal products or other pharmaceutical composition for the treatment of neurodegenerative diseases or for the treatment of diseases caused by ischaemic, traumatic or autoimmune lesions of the nervous system, or the other additional medicinal products or other pharmaceutical composition for the treatment of said diseases can be administered first, and then the pharmaceutical composition of the invention. Alternatively, any of these two components can be mixed together in the same composition and administered together. In another alternative embodiment, said pharmaceutical composition of the invention and other additional medicinal products or other pharmaceutical composition for the treatment of said neurodegenerative diseases or for the treatment of diseases caused by ischaemic, traumatic or autoimmune lesions of the nervous system, are administered simultaneously.

### Therapeutic uses of the cells and cell population of the invention

In another aspect, the invention relates to the use of said stem cells, progenitor cells or differentiated cells of the invention or combinations thereof, or of said immortalized cells of the invention, or of said cell population of the invention, in the development of a pharmaceutical composition for the treatment of neurodegenerative diseases, for example in the treatment of Alzheimer's disease, in the treatment of Parkinson's disease, in the treatment of ischaemic lesions of the nervous system, in the treatment of traumatic lesions of the nervous system, or in the treatment of autoimmune lesions of the nervous system.

In a particular embodiment, said cells are differentiated cells of the invention, or reversibly immortalized differentiated cells of the invention, or of a cell population of the invention that comprises said differentiated cells of the invention and/or said reversibly immortalized differentiated cells of the invention. Although said cells can be of autologous, allogeneic or xenogeneic origin, in a particular embodiment said cells are of autologous origin and are isolated from the CB of the subject to whom they will be administered, thus reducing potential complications associated with antigenic and/or immunogenic responses to said cells. Furthermore, in a particular embodiment, said cells are in the form of neurospheres.

In another aspect, the invention relates to a method of treatment of a disease, preferably a neurodegenerative disease or a disease caused by an ischaemic, traumatic or autoimmune lesion of the nervous system, that comprises grafting (implanting or transplanting) in the regions affected by said disease, a therapeutically effective amount of said stem cells, progenitor cells or differentiated cells of the invention or combinations thereof, or of said immortalized cells of the invention, or of said cell population of the invention, preferably differentiated cells of the invention, optionally reversibly immortalized. In a particular embodiment, said neurodegenerative disease is Alzheimer's disease or Parkinson's disease. Moreover, in a particular embodiment, said cells are glomus cells (nestin-/TH+), preferably glomus cells in the form of neurospheres.

Grafting of said cells and/or neurospheres can be carried out by techniques known in the prior art, although preferably they will be implanted by stereotaxic surgery using a rigid cannula. The localization and amount of tissue (cells and/or neurospheres) to be transplanted will depend on the type and severity of the disease. In the case of Parkinson's disease patients the cellular implants will preferably be performed bilaterally in the caudatum and/or putamen. Preferably, a minimum of 10⁶ cells, 10⁷ cells, 10⁸ cells or more, will be implanted on either side of the brain. In another embodiment, the cells may be implanted into the damaged tissue adhered to a biocompatible implant. Within this embodiment, the cells may be adhered to the biocompatible implant *in vitro,* prior to implantation into the patient. As will be clear to a person skilled in the art, any one of a number of adherents may be used to adhere the cells to the implant, prior to implantation. By way of example only, such adherents may include fibrin, one or more members of the integrin family, one or more members of the cadherin family, one or more members of the selectin family, one or more cell adhesion molecules (CAMs), one or more of the immunoglobulin family and one or more artificial adherents. This list is provided by way of illustration only, and is not intended to be limiting. It will be clear to a person skilled in the art, that any combination of one or more adherents may be used.

In another embodiment, the cells may be embedded in a matrix, prior to implantation of the matrix into the patient. Generally, the matrix will be implanted into the damaged tissue of the patient. Examples of matrices include collagen based matrices, fibrin based matrices, laminin based matrices, fibronectin based matrices and artificial matrices. This list is provided by way of illustration only, and is not intended to be limiting.

In a further embodiment, the cells may be implanted or injected into the patient together with a matrix forming component. This may allow the cells to form a matrix following injection or implantation, ensuring that the cells remain at the appropriate location within the patient. Examples of matrix forming components include fibrin glue liquid alkyl, cyanoacrylate monomers, plasticizers, polysaccharides such as dextran, ethylene oxide-containing oligomers, block co-polymers such as poloxamer and Pluronics, non-ionic surfactants such as Tween and Triton'8', and artificial matrix forming components. This list is provided by way of illustration only, and is not intended to be limiting. It will be clear to a person skilled in the art, that any combination of one or more matrix forming components may be used.

In a further embodiment, the cells may be contained within a microsphere. Within this embodiment, the cells may be encapsulated within the centre of the microsphere. Also within this embodiment, the cells may be embedded into the matrix material of the microsphere. The matrix material may include any suitable biodegradable polymer, including but not limited to alginates, Poly ethylene glycol (PLGA), and polyurethanes. This list is provided by way of example only, and is not intended to be limiting.

In a further embodiment, the cells may be adhered to a medical device intended for implantation. Examples of such medical devices include stents, pins, stitches, splits, pacemakers, prosthetic joints, artificial skin, and rods. This list is provided by way of illustration only, and is not intended to be limiting. It will be clear to a person skilled in the art, that the cells may be adhered to the medical device by a variety of methods. For example, the cells may be adhered to the medical device using fibrin, one or more members of the integrin family, one or more members of the cadherin family, one or more members of the selectin family, one or more cell adhesion molecules (CAMs), one or more of the immunoglobulin family and one or more artificial adherents. This list is provided by way of illustration only, and is not intended to be limiting. It will be clear to a person skilled in the art, that any combination of one or more adherents may be used.

Said stem cells, progenitor cells or differentiated cells of the invention as well as said immortalized cells of the invention, and said cell population of the invention, can be obtained by means of the methodology described above.

### Method for in-vitro evaluation of the cellular response to a potentially therapeutic compound

In another aspect, the invention relates to a method for *in-vitro* evaluation of the cellular response to a potentially therapeutic compound that comprises contacting a culture that contains stem cells, progenitor cells or differentiated cells of the invention or combinations thereof, and/or immortalized cells of the invention, and/or a cell population of the invention with a potentially therapeutic compound and evaluating the effects caused by said compound on the cells present in said culture.

In a particular embodiment, said cells are differentiated cells of the invention, in particular, cells differentiated to glomus cells (nestin-/TH+), optionally in the form of neurospheres.

### Use of the cells of the invention for the production of GDNF

Surprisingly, as shown in Example 7, the neurospheres generated from stem cells from the carotid body are able to produce GDNF *in vitro,* similarly to the glomus cells formed *in vivo de novo* (see Example 4), that also acquire the ability to synthesize GDNF. The production can be detected in the neurosphere region corresponding to glomus cells. Thus, the stem cells, progenitor cells or differentiated cells of the invention or combinations thereof, or a cell population of the invention, can be used for the production of GDNF.

To that aim, differentiating cells of the invention should be obtained by a method that comprises cultivating the stem cells and/or the progenitor cells of the invention under conditions that permit the differentiation of the stem cells and/or progenitor cells to the desired differentiated cells, such as culturing under a continued hypoxic stimulus; once differentiated, the cells can be cultured under normoxia for using them for the production of GDNF. Thus, based on the information provided by this description, the differentiated cells of the invention can be obtained and cultured by conventional methods known by any person skilled in the art from the CB, or from stem cells of the CB, or from progenitor cells derived from said stem cells of the CB.

It is preferred the use of cells of the invention obtained from a human being, because this could solve some of the obstacles observed during the clinical trials that have been performed to assess the usability of GDNF as a therapeutic agent for the treatment of Parkinson disease. Primate studies showed improved motor performance in the animals which received the GDNF gene (compared with animals that received no treatment). In the treated animals, parkinsonian symptoms were reduced, and, after the animals were sacrificed, the numbers of healthy dopamine neurons were found to be significantly enhanced. A summary of the findings was published in Kordower et al., Science 290, 767 (2000). Based on this and other studies conducted by Dr. Don Gash (University of Rochester and University of Kentuchy, USA) a human, randomized, double-blind trial of GDNF was initiated. The results obtained from this trial were controversial, showing little or no benefit from GDNF. The other aspect that was found during the trial was the detection of "neutralizing antibodies" in some participants. These two reasons lead the company in charge of the clinical trial to halted of the trials on GDNF on 2004. Since it has been suggested that the appearance of neutralizing antibodies and the poor results observed during the trials could be associated to the fact the used GDNF was recombinant GDNF isolated from bacteria, GDNF directly obtained from human cells could prove to be more useful as therapeutic agent for the treatment of Parkinson's disease, not giving rise to neutralizing antibodies. Thus, its production and isolation from a culture of differentiated human cells of the invention could be of use for overcoming some of the problems found in the treatment of Parkinson's disease.

The following examples illustrate the invention but must not be regarded as limiting it.

### EXAMPLES

The materials and methods that were used for the development of the present invention, as well as examples of application thereof, are detailed below. Said examples do not limit the invention - their purpose is to illustrate it, demonstrating the presence of stem cells in the carotid body, the manner of promoting their proliferation and differentiation, and the uses thereof.

### MATERIAL AND METHODS

### 1. Animals

The wild-type Wistar rats (Charles River, Barcelona, Spain), the wild-type C57BI16 mice (Charles River, Barcelona, Spain) and the transgenic mice used for these studies were kept and treated following the guidelines defined by the European Commission (861609/EEC). The transgenic mice (loxP-flanked GFAP-Cre/LacZ, GDNF/LacZ) were obtained from Jackson Laboratories (Bar Harbor, ME), whereas the loxP-flanked Wntl-Cre/LacZ mice were supplied by Dr. Andrew McMahon (Department of Molecular and Cellular Biology, Harvard University, USA). Genotyping of the transgenic animals was performed by PCR following the indications of the researchers who made them.

### 2. Treatments of chronic hypoxia in vivo and supply of BrdU

The wild-type C57BI16 mice aged 2 to 3 months were exposed chronically to an ambient atmosphere with 10% oxygen, using special hermetic chambers for this, providing control of the levels of O₂ and CO₂, and monitoring of temperature and humidity (COY Laboratory Products INC., Grass Lake, MI). To reveal the proliferation events in the carotid body, the mice were injected intraperitoneally with 50 mg/kg of 5-bromo-2-deoxyuridine [BrdU] (Sigma) every 7 days throughout the experiment. In addition, the drinking water was supplemented with 1 mg/ml of BrdU. Processing of the animals after the hypoxic/normoxic treatment comprised fixation of all the tissues by intracardiac infusion with the fixing agent paraformaldehyde (PFA) at 4% (Sigma). Next, the carotid bifurcations were removed and were post-fixed for two hours in PFA. After 12 hours of cryoprotection in 30% sucrose (Sigma), the bifurcations were embedded in the compound OCT (Sakura Finetek, Torrance, CA), a formulation based on water-soluble glycols and resins, to produce blocks and enable the bifurcations to be cut in a cryostat, to obtain slices of carotid body with a thickness of 10 microns.

### 3. Immunohistochemical analysis

To detect the BrdU by immunohistochemical analysis in the tissue sections, the DNA is denatured with 2M HCl for 45 minutes at room temperature and is then neutralized with 0.1 M sodium borate. Next, the sections are pre-blocked for 1 hour at room temperature with blocking solution (phosphate buffer plus 10% of goat serum. 0.1% of bovine serum albumin (BSA), and 0.3% of Triton X-100 (Sigma)). The primary antibody to BrdU (1:100, Accurate Chemical, Westbury, NY) was incubated on the tissue overnight at 4°C, followed by incubation for 4 hours at room temperature with the fluorescein-conjugated secondary antibody (1:200, Jackson Immunoresearch, West Grove, PA). Other antibodies used, following the same protocol as for BrdU but without the steps of denaturation/renaturation of the DNA, were: mouse anti-TH antibody produced in rabbit (1:1000, Pel-Freez, Roger, AR), and mouse anti-GFAP antibody also produced in rabbit (1:500, DAKO Cytomation, Fort Collins, CO), both followed by an anti-rabbit secondary antibody conjugated with Alexa 568 (1:500, Molecular Probes, Eugene, OR). Finally, the tissues are stained with 2.5 mg/ml of 4',6-diamidino-2-phenylindole dihydrochloride [DAPI] (Sigma) for 10 minutes at room temperature for marking the nuclei, prior to assembly with 70% of glycerol prepared in phosphate-buffered saline [PBS]. In these tests, the TH+ cells were visualized in red, the BrdU+ in green and the DAPI+ in blue.

### 4. Dissociation of the carotid bodies to single cells

The carotid bodies of 20-day-old Wistar rats were dissected and were placed in cold phosphate buffer. The glomus tissue was incubated for 20 minutes in an enzymatic solution (1 ml phosphate buffer with 0.6 mg collagenase II, 0.3 mg trypsin, 7.5 microlitres of porcine elastase (stock: 250 U/milliliti-e), and 10 microlitres of CaCl₂ from a 5 mM stock) at 37°C. Next, the glomus tissue was broken up using fine tweezers, incubated for a further 7 minutes at 37°C, and finally the cells were dispersed by pipetting them mechanically. The cell density was determined using a haemocytometer (Sigma).

### 5. Tests of growth in vitro in the form of neurospheres

The dispersed carotid body cells were cultivated typically in 6-well plates treated for non-adherence (Corning Inc., Corning, NY), at a clonal density to promote the formation of individually identifiable neurospheres. For the experiments of clonal growth and individualization, plates treated for non-adherence were used, but with 96 wells instead of 6. The culture medium contained: D-MEM:F-12 (Gibco BRL, Grand Island, NY), 15% of chick embryo extract (prepared as described in Stemple and Anderson, 1992; Cell 71, 973-985), 1% of N₂ supplement (Gibco), 2% of B27 supplement (Gibco), 1% of penicillin/streptomycin (Cambrex, Verviers, Belgium), 20 ng/ml of human recombinant basic fibroblast growth factor (bFGF) (R&D Systems, Minneapolis, MN), 20 ng/ml of human recombinant insulin-like growth factor-1 (IGF-1) (R&D Systems), and 20 ng/ml that contained basic fibroblast growth factor (bFGF), insulin-like growth factor-I (EGF-1) and human recombinant epidermal growth factor (EGF) (R&D Systems). Throughout this work, this composition of medium is called neural crest culture medium (NC medium). All the cultures were conducted in incubators (Thermo Electron Corp., Waltham, MA) with controlled CO₂ level, at fixed oxygen level of 3% and at 37°C.

For testing the capacity for self-renewal of the stem cells from the carotid body, individual primary neurospheres were used, they were re-plated on adherent substrate treated with poly-L-lysine, and were incubated for 48 hours with NC medium. Next, they were detached with trypsin and were dispersed mechanically, finally cultivating the cells again on non-adherent plates with NC medium. The secondary neurospheres were counted after 10 days of culture.

For immunocytochemical identification of the various cells within the neurospheres, these were fixed in 4% PFA and the same protocol was applied to them for obtaining thin slices as for the complete carotid bodies (cryoprotection, embedding, and fine cutting of 10 microns).

On other occasions, the neurospheres were re-plated on plates treated for adherence, incubated for 10 days with NC medium, and once the colonies had expanded, were fixed with 4% PFA. Then a typical immunocytochemical protocol was applied: preblocking for 1 hour at room temperature, incubation with the primary antibody for 1 hour, and with the secondary antibody for one more hour, and finally contrastaining with DAPI, which stains the cell nuclei blue. The blocking solution was the same as was used for the tissues (see above). The antibodies used in the samples in conditions of adherence were: anti-TH antibody produced in mouse (1:500, Sigma), followed by an anti-mouse secondary antibody produced in goat and conjugated with rhodamine (1:100, Chemicon International. Temecula, CA), and anti-smooth muscle actin antibody (SMA, Sigma), followed by an anti-mouse secondary antibody produced in donkey and conjugated with Cy2 (1:200, Jackson Immunoresearch). The antibodies used on thin sections of neurospheres were: mouse anti-TH antibody produced in rabbit (the same as for the tissues, see above) and rat anti-nestin antibody produced in mouse (1:200, Chemicon International) followed by an anti-mouse secondary antibody produced in donkey and conjugated with Cy2 (1:200, Jackson Immunoresearch). The nestin-positive cells were visualized green.

### 6. Flow cytometry

All the analyses and cell separations by cytometry were carried out in a three-laser flow cytometer model MoFlo (DAKC) Cytomation, Fort Collins, CO). The dispersed carotid body cells were resuspended in staining solution and were incubated with the various antibodies, each for 20 minutes at 4°C. The staining solution contained (for 50 ml): 44 ml of L15 medium (Gibco), 0.5 ml of penicillin/streptomycin (Cambrex), 0.5 ml of HEPES 1 M buffer (Gibco), 0.1 g of BSA (Sigma), and 5 ml of distilled and deionized water. The antibodies used for flow cytometry were as follows: mouse anti-p75 antibody produced in rabbit (1:2000, Chemicon International) followed by an anti-rabbit secondary antibody produced in goat and conjugated with fluorescein-5-isothiocyanate [FITC] (1:200, Jackson Immunoresearch), and mouse anti-HNK-1 (1:600, Pharmingen/BD Biosciences, San Jose, CA) followed by an anti-mouse secondary antibody produced in goat and conjugated with phycoerythrin [PE] (1:200, Jackson Immunoresearch). Once the remaining, non-adhering antibodies had been washed, the cells were resuspended in staining medium with 2 mg/ml of 7-amino-actinomycin D [7-AAD] (Molecular Probes, Eugene, OR). The dead cells were eliminated in the cytometer by their 7-AAD positive phenotype.

### 7. Amperometric recording of cellular secretion

Some neurospheres grown from the carotid body were replated on cover slips treated with poly-L-lysine, and were incubated in normal culture medium overnight. The cover slips with the adhering neurospheres were transferred to a recording chamber and were perfused continuously with a solution containing NaCl 117 mM, KCl 4.5 mM, NaHCO₃ 23 mM, MgCl₂ 1 mM, CaCl₂ 2.5 mM, glucose 5 mM and sucrose 5 mM. The "hypoglycaemic" solution had sucrose 5 mM instead of glucose, i.e. total sucrose 10 mM, to prevent changes in osmolality. The "normoxic" solution was bubbled with a gas mixture of 5% CO₂, 20% O₂, and 75% N₂ (O₂ pressure 145 mmHg). The "hypoxic" solution was bubbled with 5% CO₂ and 95% N₂ to reach an O₂ pressure in the chamber of approximately 15 mmHg. All the experiments were carried out at a temperature in the chamber of approx. 36°C. The rate of secretion, in femtocoulombs (fC)/minute, was calculated as the amount of charge transferred to the recording electrode in a given time (Pardal and López-Barneo, 2002; Nat Neurosci 5, 197-198).

### 8. Recording by the "patch clamp" technique

The voltage-dependent macroscopic flows of glomus cells in the glomeruli of the neurospheres were recorded using the "complete cell" configuration of the "patch clamp" technique,as previously adapted in the laboratory of the inventors (López-Barneo et al., 1988; Science 241, 580-582). For recording the macroscopic flows of potassium, the composition of the recording solutions was: internal: 80 potassium glutamate 80 mM, KCl 50 mM, MgCl₂ 1 mM, Hepes 10 mM, MgATP 4 mM, ethylene glycol tetraacetic acid [EGTA] 0.1 mM (pH, 7.2); and external: NaCl 117 mM, KCl 4.5 mM, NaHCO₃ 23 mM, MgCl₂ 1 mM, CaCl₂ 2.5 mM, glucose 5 mM and sucrose 5 mM (pH adjusted by bubbling with 5% CO₂) The potential fixed at rest was -80 mV. For the experiments for recording macroscopic flows of calcium the composition of the solutions was: mternal:CsCl 100 mM, CsF 25 mM, MgCl₂ 2 mM, Hepes 10 mM, MgATP 4 mM, EGTA 0.5 mM (pH 7.2); and external: BaCl₂ 10 mM, NaCl 140 mM, KCl 2.7 mM, Hepes 10 mM and glucose 5 mM (pH 7.4). The holding potential at rest was -80 mV.

### 9. Statistical analysis

The statistical data are shown as the mean ± standard deviation, with the number of repetitions of the experiment in parentheses. The statistical significance was estimated by a matched pairs Student *t*-test.

### 10. Obtaining neurospheres of human carotid body

The two carotid bifurcations were received from heart donors under 60 years of age. The carotid bodies were dissected from the carotid arteries under a microscope, with the tissue cold all the time and immersed in phosphate buffer at pH 7.4. Once extracted, the carotid bodies were cut into small fragments that were submitted to enzymatic dispersion immediately. The dispersion solution consisted of 6 ml of phosphate buffer with 60 µl of CaCl₂ (stock 5 mM), 1.6 mg trypsin, 2.5 mg collagenase type I, 2.5 mg collagenase type IV and 7.5 µl porcine elastase. The human glomus tissue was incubated in the dispersion solution for 20 minutes at 37°C and with agitation. Then the tissue was broken up using fine tweezers, incubated again at 37°C for a further 10 minutes, and finally the cells were mechanically dispersed by pipetting the tissue. Once dispersed, the cells were plated on low-adherence plates with culture medium that contained the same as for rodent cells, but replacing the chick serum with human serum obtained following a standard haematological method from peripheral blood from volunteer donors (name of the kit: Vacuette Z serum SEP clot activator). After 10 days in culture, the human neurospheres were photographed and were processed in the same way as those from rodents for immunohistochemical studies.

### 11. Staining with X-gal

The dispersed cells, sections of tissues or sections of neurospheres were stained for detecting the expression of β-galactosidase, incubating them overnight at 37°C in buffer with X-gal. The buffer was prepared with PBS (pH=7.4) supplemented with MgCl₂ 2 mM (Sigma), potassium ferrocyanide 5 mM (Sigma) and potassium ferricyanide (Sigma). X-gal (Molecular Probes, Eugene, OR) was added to the buffer just before use, to a final concentration of 1 mg/ml.

### EXAMPLE 1

### Induction of growth of the carotid body by hypoxia and generation of new glomus cells derived from the neural crest

Exposure of wild-type C57BL/6 mice to hypoxia (10% O₂) in an isobaric atmosphere for 3 weeks induced an increase in size of the carotid body of approximately 2 to 3 times relative to the initial size, owing to dilatation and multiplication of blood vessels, as well as expansion of the parenchyma, with an increase in the number of TH+ glomus cells (positive for tyrosine hydroxylase) (Fig. 1A).

For analysis of the proliferation and formation of new glomus cells, the C57BL/6 mice were treated with BrdU and were then kept for several days in a normoxic (21% O₂) or hypoxic (10% O₂) environment. No TH+ and BrdU+ cells were observed in the normoxic animals, indicating absence of proliferation (Fig. 1B, left). In contrast, after some days in hypoxia, even before growth of the CB was apparent, numerous TH+ and BrdU+ cells were observed (arrows in Fig. 1B right), indicating the appearance of new glomus cells. The changes produced in the CB in response to hypoxia are shown as a function of time in Fig. 1C. Parallel to hypertrophy of the CB, the number of TH+ glomus cells formed *de novo* during the treatment under conditions of hypoxia also increased by a factor of 2-3.

This quantitative analysis also revealed that, whereas incorporation of BrdU in the tissue of the CB was observed from day 1 after application of the conditions of hypoxia, the new glomus cells (BrdU+, TH+) only start to appear after 5 or 6 days in hypoxia. These data would suggest the existence of a process of proliferation and differentiation prior to appearance of the new neuronal cells. Moreover, when the mice treated chronically with hypoxia were re-introduced into a normoxic environment (renormoxia), recovery of normal size of the CB proceeded in parallel with the decline in the number of glomus cells. Thus, in the re-normoxic CB approximately 50% of the glomus cells were BrdU+ (Fig. 1D-F, arrows). These data indicate once again the existence of precursor or progenitor cells (BrdU+, TH-), whose proliferation (incorporation of BrdU) in hypoxia precedes their differentiation to glomus cells (TH+). Moreover, it was observed that the structural modification induced by hypoxia was similar both in young mice (4-6 weeks, Fig. 1C) and in older mice (over 8 months; TH+ cells: 1555+25, n=4 in normoxia and 2470±358, n=3 in hypoxia).

The neural origin of the precursor cells that give rise to the glomus cells was tested by analysis of the cell lineage, using the loxP-flanked Wnt1-Cre/LacZ transgenic mouse, in which the cells derived from the neural crest are marked (Chai et al. 2000 Development 127, 1671-1679; Jiang et al., 2000 Development 127, 1607-1616) (Fig. 2A). The CB in these animals contains a large amount of deposits of X-gal that are co-localized with TH+ cells. Precipitates of X-gal were also observed in BrdU+, TH+ cells (Fig. 2B-C). These data show that both the pre-existing glomus cells and those newly formed in animals subjected to hypoxia originate from the neural crest.

### EXAMPLE 2

### Identification of self-renewing and pluripotent stem cells of the carotid body

For identification of the precursors that give rise to the glomus cells in adult rodents exposed to hypoxia, the carotid bodies of young Wistar rats (>P20) were dispersed enzymatically and the cells were plated in neural crest (NC) culture media that contained bFGF, 1GF-1 and EGF (Morrison et al., 1999, Cell 96, 737-749). Using culture plates treated for non-adhesion, growth was induced in conditions of flotation and neurospheres were thus obtained. Clonogenic proliferation was stimulated by culture of the cells in moderate hypoxia (3% O₂) (Morrison et al., 2000, J Neurosci 20, 7370-7376), a condition that mimics the stimulation by hypoxia of the growth of the CB in *vivo.*

In these experiments, 1.04±0.4% (n=6 preparations) of the cells plated gave rise to neurospheres (Fig. 3A). After 10 days in culture the diameter of the neurospheres was 85±34 micrometres (n=112), a value comparable to that described for neurospheres obtained from other neural progenitor populations (Molofsky et al. 2003; Nature 425, 962-967). Growth of the neurospheres requires the presence of growth factors in the culture medium for NC (NC medium) and moreover, the neurospheres obtained are noticeably smaller if culture is carried out in conditions of normoxia (21% O₂). Furthermore, it is also observed that the efficiency of formation of neurospheres decreases to 0.17±0.02% (n=3 preparations) in old animals.

However, compared with the typical spherical shape of the neurospheres derived from the central nervous system (CNS) and other areas of the peripheral nervous system (PNS) (Doestsch et al., 1999, Cell 97, 703-716; Molofsky et al., 2003, Nature 425, 962-967) the majority (>85% at ten days of culture) of the neurospheres derived from the CB had characteristically one or two protrusions (arrows in Fig. 3A). Immunocytochemical analysis of thin sections of neurospheres revealed the presence of nestin (nestin+) (typical marker of neural progenitor cells) in the central core, and aggregates of differentiated TH+ and nestin- cells in the glomerular protrusions that are joined to the central core via a narrow filament of tissue (Fig. 3B), and in their form are reminiscent of the glomeruli characteristic of the CB *in situ.* After several weeks in culture, these glomeruli grew until they reached the size of an adult CB (Fig. 3C).

The clonal origin of the neurospheres was confirmed by experiments with individual plating of cells, using 96-well plates with ultralow adherence. After 10 days in culture, numerous wells that received a single cell, checked twice under the microscope, had a single neurosphere with glomeruli, indicating that these neurospheres arose from the proliferation of just one cell originating from the CB. The estimated efficiency in the formation of neurospheres was 11.6±1.5% (experiments n=4), significantly greater than the values obtained in the experiments with culture of all of the cells. This difference may simply be due to reduced survival of the cells in culture in the experiments with plating of all of the cells, owing to the presence of dead cells and cellular residues. In any case, the estimated frequency of formation of neurospheres suggests that there should be of the order of 200 to 300 stem cells in a normal rat CB.

Moreover, the capacity for self-renewal of the progenitor cells of the CB was also tested, by dissociation of the primary neurospheres and re-plating, on non-adherent dishes with NC medium, of the cells dispersed in culture in the same conditions. The number of secondary neurospheres obtained (7±2 per primary neurosphere, n=10) was sufficiently less than the values described in the literature in relation to other populations of neural progenitors (Molofsky et al., 2003 Nature 425, 962-967). However, the difficulties encountered during the process of dissociation of the neurospheres of the CB, requiring strong enzymatic treatment and a vigorous disruption process, suggest that the cells may have suffered damage and, consequently, their secondary growth is altered. In this sense, it is not possible to rule out a greater capacity for self-renewal of the progenitor cells of the CB if the tests are carried out in better conditions.

The presence of TH-, nestin- cells in the central core of the large neurospheres (Fig. 3B and 3C) suggests that, as well as the glomus cells, the progenitor cells of the CB can differentiate to other cellular types. This was verified by immunocytochemical analysis in neurospheres re-plated in culture on an adherent substrate. The stem cells of the CB were capable of differentiating to SMA+ cells (specific antigen of smooth muscle actin), a cellular type typically derived from the neural crest in culture (Fig. 3D and 3E) (Kruger et al., 2002, Neuron 35, 657-669). GFAP+ staining does not appear in the neurospheres, since, as was shown in the experiments *in vivo* (see Example 3), the cells lose this marker at the moment when they begin the proliferative process. Thus, the progenitors of the CB behave similarly to other stem cells derived from the neural crest (Bixby et al., 2002, Neuron 35, 643-656), being capable of production of clonogenic colonies and self-renewal and differentiation to multiple cellular types.

### EXAMPLE 3

### The sustentacular cells of glial type are the stem cells of the CB

Until now, it has always been thought that in certain circumstances the glomus cells are capable of entering a mitotic cycle (Nurse and Vollmer, 1997 Dev Biol 184, 197-206; Wang and Bisgard, 2002 Microsc Res Tech 59, 168-177). However, it is well established that the TH+ glomus cells can, after dissociation, be maintained in culture for several days (López-Barneo et al., 1988 Science 241, 580-582; Villadiego et al., 2005 J Neurosci 25, 4091-4098). To verify whether proliferation of the glomus cells gave rise to the CB neurospheres, an immunocytochemical analysis was carried out in different stages of formation of neurospheres (Fig. 4A). Initially, the neurospheres were small and were formed by groups of TH-, nestin+ and other nestin- cells. This stage is soon followed by the appearance of small germinal nuclei (protrusions) with some TH+ cells at the periphery of the growing neurospheres. The number of TH+ cells increases progressively until they form the characteristic aggregate of glomus cells described previously. This sequential development suggests that after dissociation and plating, the progenitor cells (different from the mature glomus cells) proliferate and undergo self-renewal to form the central core of the neurospheres, which precedes the differentiation of the progenitors to mature glomus cells (TH+).

Separation by flow cytometry suggested that the TH+ cells do not initiate the formation of neurospheres, since the cells that express a large amount of HNK-1, a marker of neuronal precursors derived from the neural crest (window #1 in Fig. 4B) (Young et al., 1998 Dev Biol 202, 67-84), never produce neurospheres. As shown in Fig. 4C the populations enriched owing to the marker HNK-1 were all TH+ glomus cells.

However, separation of a fraction of cells that strongly expressed p75, a typical marker of other populations of stem cells from the neural crest (Morrison et al., 1999, Cell 96, 737-749) (window #2 in Fig. 4B), did not increase the production of neurospheres. Other analyses with markers of stem cells (c-ret, CD133, integrin alpha-4 and SSEA-1) also proved unsuccessful for enriching the populations of progenitors from CB by flow cytometry. A clue to the identity of the stem cells came from the experiments conducted *in vivo* that showed that whereas in the CB of normoxic animals the type II cells were clearly identified with the GFAP marker (GFAP+), said marker disappeared progressively with exposure to hypoxia, at the same time as the number of BrdU+ or proliferative cells increased (Fig. 4D). Moreover, on bringing the type II cells to a hypoxic environment there is also appearance of the proliferation marker nestin, which disappears on return to normoxic environments (Fig. 9 A-D). The GFAP+ cells reappeared once the hypoxic animal returned to a normoxic atmosphere and the CB returned to its original size. These data suggested that the type II cells were undergoing a process of activation in response to hypoxia and could therefore be the stem cells of the CB.

Direct evidence of the identity of the stem cells of the CB was obtained by experiments *in vivo* for mapping the cell lineage, using a loxP-flanked GFAP-Cre/LacZ transgenic mouse (Fig. 5A). In these animal models the transgenic promoter of GFAP is fairly active in the brain, where many cells (glial cells and neurons) originate from GFAP+ progenitors and, consequently, all appear marked as B-gal+ (Malatesta et al., 2003, Neuron 37, 751-764). In contrast, the activity of the transgenic promoter of GFAP was fairly low in normoxia in the CB and, moreover, B-gal+ cells were not detected in the CB of animals that had never been submitted to conditions of hypoxia. For activation of the transgenic promoter of GFAP, the animals were subjected to cycles of hypoxia/renormoxia, causing disappearance and reappearance of expression of GFAP in the sustentacular cells and activation of the transgenic construction. In these animals, a second exposure to hypoxia produced growth of the CB and selective deposition of X-gal in the new glomus cells (TH+ and BrdU+). This demonstrates that they are derived from GFAP+ progenitors. The localization of the deposits of X-gal on the BrdU+, TH+ cells was confirmed by dispersion of cells and staining of isolated cells.

It can be seen from Fig. 9 that the type II GFAP+ cells of the CB that display deposits of X-gal are the stem cells, since they give rise to a growth in the form of neurospheres (TH+ glomus cells).

### EXAMPLE 4

### The glomus cells generated in vitro have mature chemoreceptor, electrophysiological and neurochemical characteristics

Glomus cells are physiologically very complex, since they are electrically excitable and they are able to detect certain physicochemical variables in blood. When activated by the drop in concentrations of O₂ or of extracellular glucose, the glomus cells of the CB exhibit a sharp increase in the release of catecholamines, which can be monitored by amperometry (Pardal et al., 2000 Proc Natl Acad Sci USA 97, 2361-2366, Pardal and López-Barneo, 2002 Nat Neurosci 5, 197-198). These same responses to hypoxia and hypoglycaemia were detected in glomus cells obtained in *vitro* from stem cells or progenitor cells of the invention (Fig. 6 A-C).

The glomus cells of the glomeruli generated *in vitro* were analysed by the "patch clamp" technique. Fig. 6D shows that the new glomus cells show inward ionic currents followed by large outward currents (Fig. 6E). As happens with normal glomus cells, blockade of the outward currents with Cs⁺ reveals the presence of voltage-gated Ca²⁺ channels (Fig. 6F). These data indicate that the TH+ cells derived *in vitro* from progenitors of the CB have the same functional characteristics as the mature glomus cells.

However, as well as being highly dopaminergic, the glomus cells of the CB are able to produce large amounts of GDNF, a potent neurotrophic factor that promotes survival of the dopaminergic neurons. In the heterozygotic GDNF/LacZ *knock out* mice the production of GDNF is marked (Sanchez et al., 1996 Nature 382, 70-73) by the appearance of deposits of X-gal that appear selectively concentrated in the glomus cells. Using this same tool, the experiments showed that the exposure of the aforementioned mice to hypoxia gave rise to deposits of X-gal on TH+, BrdU+ cells in the CB *in vivo* (Fig. 7A-D). These data indicate that the glomus cells formed *de novo* also acquire the ability to synthesize GDNF.

### EXAMPLE 5

### Applicability to cell therapy in Parkinson's disease

The model of Parkinson's disease in Wistar rats based on unilateral lesion of the substantia nigra by a toxic substance, 6-hydroxydopamine (6-OHDA), that selectively destroys the dopaminergic neurons, was used for the experiments. Rats with a lesion greater than 90% of the substantianigra presented symptoms that resemble Parkinson's disease in humans: akinesia, rigidity, lateralization of movements (rotation in the rat) and sensorimotor deficit. These functional defects could be evaluated with a suitable battery of tests. Furthermore, the lateralization of movements could be exacerbated by injection of amphetamine (5 mg/kg), which induced intense "homolateral rotation" (towards the side with the lesion), whose frequency, if greater than 360 turns per hour, was indicative of a lesion greater than 90%.

For each parkinsonian rat, about 20 neurospheres from carotid body were implanted in the denervated striatum (coordinates AP=0.2; L=-3; V=-5.5 relative to bregma), ten days from unilateral lesion of the substantianigra with 1 microlitre of 6-OHDA (4 mg/ml; coordinates AP=-5.3; L=-2.3; V=-8.2). The carotid body neurospheres were obtained after dispersion and dissociation of the carotid body by incubation for 20 minutes in physiological solution without calcium or magnesium but with collagenase (0.6 mg/ml) and trypsin (0.3 mg/ml). The dispersed cells were plated on non-adherent plates with culture medium that contained trophic factors specific to stem cells derived from the neural crest. Each neurosphere obtained by this method contained between 50 and 100 glomus or pei-fectly differentiated type I cells.

At ten, thirty and ninety days after transplant of the neurospheres, the functionality and efficacy of the graft were evaluated from a neurochemical, morphological and behavioural standpoint. The neurochemical investigation was based on amperometric recording of the intrastriatal dopamine levels in slices of brain tissue. The morphological investigations were of an immunocytochemical character, using staining with antibodies to tyrosine hydroxylase (TH), which indicated the presence or absence of dopamine producing cells. The behavioural evaluation was carried out using a suitable battery of tests, for evaluating the animal's motor and sensorimotor responses.

The functional results were very positive: the amperometric recordings, which detected a drop greater than 90% of dopamine in the denervated striatum without transplant, revealed a recovery to 65% of the dopamine levels in the striatum with transplant of three months of evolution. The morphological studies showed the presence of transplants with glomus cells positive to tyrosine hydroxylase, associated in glomeruli and that also expressed trophic factors of the GDNF type (Fig. 7). The foregoing correlated with a very good behavioural recovery: a) rotation, both spontaneous and amphetamine-induced, disappeared during the first month, and b) the sensorimotor reflexes were restored after three months.

### EXAMPLE 6

### Identification of stem cells in the human carotid body

To corroborate the existence of stem cells from the carotid body in human tissue, the authors enrolled in the Organ Donations programme of the Virgen del Rocío Hospital in Seville. They thus began to receive the two carotid bifurcations (Fig. 11A) of heart donors, which display better preservation of the vascular tissue, under 60 years of age. The carotid bodies (Fig. 11B) were dissected and sliced (Fig. 11C) and then underwent an enzymatic treatment. The dispersed cells were cultivated in neural crest medium with human serum and in hypoxic conditions. After 10 days in culture, the existence of neural progenitors was observed, growing in the form of neurospheres (Fig. 11D), very similar in appearance to those described in rodents, that is, with thickenings present on the surface (arrowheads in Fig. 11D) in the manner of glomeruli. Detailed immunocytochemical investigation of these neurospheres revealed that the thickenings were effectively groups of TH+ glomus cells (Fig. 11E), that were differentiating starting from the progenitors. These findings therefore confirm the existence of stem cells specific to the carotid body in humans, and show their capacity for differentiation to glomus cells in vitro.

### EXAMPLE 7

### Demonstration of the production of GDNF bv the neurospheres

As already mentioned, the stem cells from the carotid body are cells capable of differentiating to mature glomus cells of the carotid body, positive for TH, and capable of secreting, in response to physiological stimuli, catecholamines such as dopamine and trophic factors such as GDNF. To confirm that this is so, a test was carried out to verify whether the neurospheres are able to produce GDNF.

For this, carotid bodies were removed from GDNF/LacZ transgenic mice (which are mice that express β-galactosidase under the control of the GDNF promoter), were dissected and the cells were dispersed in a manner similar to that described for Wistar rats, cultivating them *in vitro* to obtain neurospheres. The neurospheres were replated on plates treated for adherence, as described in the section "Tests of growth *in vitro* in the form of neurospheres" and then the immunocytochemical test protocol described in this same methodology section was applied to the plates. In addition, a process of staining with X-gal was carried out on the plates, for verifying expression of the enzyme β-galactosidase.

The test results are presented in Fig. 12, in which it is shown that appearance of the deposits of X-gal (panel A) occurs in the region in which the fluorescence due to the anti-TH antibodies, the region of the glomus cells, is detected. The density of the points, moreover, correlates with a high level of production of GDNF in the stained cells, demonstrating the production of GDNF *in vitro* in the neurospheres generated from stem cells from the carotid body.

### CONCLUSIONS

Although it was known that neurogenesis is maintained in specific niches of the brain of adult mammals, the existence of germinal centres in the PNS was unknown. Now, the inventors have discovered, surprisingly, the presence of stem cells in the CB of adult mice. The research undertaken has demonstrated that production *de novo* of neuron-like glomus cells depends on a population of stem cells present in the CB, which form pluripotent, self-renewing colonies *in vitro.* Analyses of the cell lineage *in vivo* indicate that, unexpectedly, the stem cells of the CB are sustentacular cells or type II cells with glial phenotype.

Autografts performed to date with cellular aggregates of the carotid body have alleviated the symptoms of Parkinson's disease patients (Arjona et al., 2003), but it was observed that there are factors that limit this therapy, that reside in the histological integrity of the CB in old age, as well as the scant amount of tissue available for the transplants. The experiments conducted by the inventors show how, after 10 days in culture, the stem cells and/or the progenitor cells of the CB of adult rodents, treated by the method of expansion of the invention, are able to produce glomus cells very efficiently.

Furthermore, culture over aggregates of glomus cells for more than three weeks in a single neurosphere is capable of reaching the size of a complete CB, so it becomes possible to obtain a large amount of cellular biomass for use in therapeutic treatments. Moreover, these new cells have the same chemosensor and electrophysiological properties as the adult glomus cells *in situ,* (Fig. 6), are highly catecholaminergic, synthesize trophic factors such as GDNF (Fig. 7) and have produced very positive results in cellular therapy against Parkinson's disease.

Thus, the tests performed have established that:
a) the CB is a germinal centre of the PNS, where the neurogenesis of adult neuronal cells takes place *in vivo,* just as occurs in the CNS of mammals, where the adult neural tissues retain progenitors that produce new neurons throughout the animal's life;
b) the germinal centres of the CB have an outstanding capacity for regeneration *in vivo*, with an important physiological role during adaptation to chronic hypoxia;
c) the CB contains pluripotent stem cells with capacity for self-renewal (type II cells), and capacity for differentiation, *in vivo* and *in vitro,* to type I or glomus cells; and
d) transplants of the cells obtained by the method of expansion of the invention are capable of reversing the clinical features of neurodegenerative diseases.

### Embodiments of the invention

1. Isolated adult stem cell, originating from the carotid body, **characterized in that** it is positive for the phenotypic marker GFAP (glial fibrillary acidic protein) and negative for the phenotypic markers TH (tyrosine hydroxylase) and nestin.
2. Isolated adult progenitor cell, derived from said stem cell according to embodiment 1. **characterized in that** it is positive for the phenotypic marker nestin.
3. Progenitor cell according to embodiment 2, **characterized in that** it is weakly positive for the marker GFAP and positive for the phenotypic marker nestin.
4. Progenitor cell according to embodiment 2, **characterized in that** it is negative for the marker GFAP and positive for the phenotypic marker nestin.
5. Differentiated adult cell, derived from said stem cell according to embodiment 1 or from said progenitor cell according to any of embodiments 2 to 4, **characterized in that** it is negative for the phenotypic marker nestin and positive for a specialization marker.
6. Differentiated cell according to embodiment 5, **characterized in that** said specialization marker is tyrosine hydroxylase (TH).
7. Differentiated cell according to embodiment 5, **characterized in that** said specialization marker is smooth muscle actin (SMA).
8. Cell according to any of embodiment 1 to 7, **characterized in that** it has been manipulated genetically.
9. Reversibly immortalized cell, **characterized in that** said cell is selected from the group comprising a stem cell according to embodiment 1, a progenitor cell according to any of embodiment 2 to 4, and a differentiated cell according to any of embodiment 3 to 5.
10. Cell according to any of embodiment 1 to 8, of human origin.
11. Isolated population of cells derived from the carotid body that comprises a set of cells according to any of embodiment to 10 or combinations thereof.
12. Pharmaceutical composition that includes a therapeutically effective amount of cells according to any of embodiment 1 to 10 or combinations thereof, or of a cell population according to embodiment 11, and a pharmaceutically acceptable excipient.
13. Use of cells according to any of embodiment 1 to 10 or combinations thereof, or of a cell population according to embodiment 11, in the development of a pharmaceutical composition for the treatment of a neurodegenerative disease, in the treatment of ischaemic lesions of the nervous system, in the treatment of traumatic lesions of the nervous system, or in the treatment of autoimmune lesions of the nervous system.
14. Use according to embodiment 13, in which said neurodegenerative disease is Alzheimer's disease or Parkinson's disease.
15. Use according to either of embodiment 13 or 14, in which said cells are glomus cells (nestin-/TH+) *de novo*, derived from said stem cell according to embodiment 1 or from said progenitor cell according to any of embodiments 2 to 4.
16. Use according to embodiment 15, in which said glomus cells *de novo* are within neurospheres.
17. Method for the preparation or expansion of stem cells according to embodiment 1 or of progenitor cells according to any of embodiments 2 to 4, that comprises cultivating said stem cells or progenitor cells under conditions of hypoxia in a culture medium that is specific for the neural crest.
18. Method according to embodiment 17, **characterized in that** said stem cells or progenitor cells are cultivated on a non-adherent surface.
19. Method according to embodiment 17, **characterized in that** said culture medium that is specific for the neural crest comprises a culture medium, a source of nutrients, antibiotics and a growth factor.
20. Method according to embodiment 19, **characterized in that** said growth factor is selected from a fibroblast growth factor (FGF) or a functional variant thereof, an insulin-like growth factor-I (IGF-I) or a functional variant thereof, an epidermal growth factor (EGF) or a variant thereof, and a combination of said factors.
21. Method according to embodiment 17, **characterized in that** culture of said stem cells or progenitor cells is carried out in the presence of an oxygen concentration of less than 15%, preferably between 1-10%, more preferably between 2-5% and even more preferably 3%.
22. Method for *in-vitro* evaluation of the cellular response to a potentially therapeutic compound that comprises contacting a culture that contains cells according to any of embodiments 1 to 10, with a potentially therapeutic compound and evaluating the effects caused by said compound on the cells present in said culture.
23. Use of cells according to any of embodiments 1 to 10 or combinations thereof, or of a cell population according to embodiment 11, for the production of GDNF.

## Claims

1. Differentiated adult cells, wherein the differentiated cells are derived *in vitro* from an isolated adult stem cell, originating from the carotid body, **characterized in that** they were positive for the phenotypic marker GFAP (glial fibrillary acidic protein) and negative for the phenotypic markers TH (tyrosine hydroxylase) and nestin.

2. Differentiated cells according to claim 1, wherein the differentiated cells are glomus cells (nestin-/TH+) *de novo.*

3. Differentiated cells according to claim 2, wherein the glomus cells (nestin-/TH+) *de novo* are in the form of glomeruli.

4. A neurosphere, wherein the neurosphere is derived *in vitro* from an isolated adult stem cell, originating from the carotid body, **characterized in that** they were positive for the phenotypic marker GFAP (glial fibrillary acidic protein) and negative for the phenotypic markers TH (tyrosine hydroxylase) and nestin.

5. The cells according to any one of claims 1 to 3, or a neurosphere according to claim 4 for use in treating neurodegenerative disease, ischaemic lesions of the nervous system, traumatic lesions of the nervous system, or autoimmune lesions of the nervous system.

6. The cells or neurosphere according to claim 5, wherein said neurodegenerative disease is Alzheimer's disease or Parkinson's disease.

7. A method for producing a trophic factor, using a cell selected from the group consisting of: (i) an adult stem cell isolated from the carotid body **characterized in that** the cell is positive for the phenotypic marker GFAP (glial fibrillary acidic protein) and negative for the phenotypic markers TH (tyrosine hydroxylase) and nestin; (ii) an adult progenitor cell, isolated from the carotid body **characterized in that** it is negative for the phenotypic marker GFAP and positive for the phenotypic marker nestin; (iii) an adult progenitor cell, isolated from the carotid body **characterized in that** it is weakly positive for the phenotypic marker GFAP and positive for the phenotypic marker nestin; or (iv) a nestin-/TH+ cell derived from a cell according to any one of the cells defined in i to iii.

8. A method for producing a trophic factor, the method comprising the steps of: (i) isolating cells from the carotid body; (ii) selecting stem cells that are (a) positive for the phenotypic marker GFAP and negative for the phenotypic markers TH and nestin; and/or (b) selecting adult progenitor cells that are positive for the phenotypic makers GFAP and nestin and negative for the phenotypic marker TH; (iii) cultivating said stem cells on a non-adherent surface under conditions of hypoxia in a culture medium that is specific for the neural crest, wherein said specific medium comprises human recombinant bFGF, human recombinant IGF-1, and human recombinant EGF.

9. A method according to claim 8 further comprising the step of culturing said stem cells under conditions that permit differentiation.

10. A method according to any one of claims 8 or 9 comprising the step of culturing the cells under a continued hypoxic stimulus.

11. A method according to any one of claims 8 to 10 comprising the step of culturing the cells under conditions of normoxia.

12. A method according to any one of claims 8 to 11 comprising the step of culturing the cells under conditions of hypoglycaemia.

13. A method according to any one of claims 8 to 12 wherein the culture conditions permit differentiation of cells into nestin-/TH+ glomus cells.

14. A method according to any one of claims 8 to 13 wherein the culture conditions permit differentiation of cells into neurospheres.

15. A method according to any one of claims 8 to 14 further comprising the step of isolating the trophic factor from the culture media.

16. A method according to any one of claims 7 to 15 wherein the trophic factor is GDNF.
